(19) Europäisches Patentamt
European Patent Office
Office européen des brevets

(11) EP 3 892 721 A1

(12) **EUROPEAN PATENT APPLICATION**
published in accordance with Art. 153(4) EPC

(43) Date of publication:
**13.10.2021 Bulletin 2021/41**

(21) Application number: **19885404.4**

(22) Date of filing: **13.11.2019**

(51) Int Cl.:
***C12N 5/0783*** *(2010.01)*

(86) International application number:
**PCT/KR2019/015469**

(87) International publication number:
**WO 2020/101361 (22.05.2020 Gazette 2020/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.11.2018 KR 20180139722**

(71) Applicant: **Green Cross Lab Cell Corporation
Yongin-si, Gyeonggi-do 16924 (KR)**

(72) Inventors:
- **KIM, You-Sun
Gyeonggi-do 16924 (KR)**
- **KIM, Eun Ji
Gyeonggi-do 16924 (KR)**
- **PARK, Gyeong Min
Gyeonggi-do 16924 (KR)**
- **YANG, Bitna
Gyeonggi-do 16924 (KR)**
- **MIN, Bokyung
Gyeonggi-do 16924 (KR)**
- **CHO, Sungyoo
Gyeonggi-do 16924 (KR)**
- **HWANG, Yu Kyeong
Gyeonggi-do 16924 (KR)**

(74) Representative: **Hughes, Sean David et al
Schlich
9 St. Catherine's Road
Littlehampton, West Sussex BN17 5HS (GB)**

(54) **METHOD FOR CULTURING CORD BLOOD-DERIVED NATURAL KILLER CELLS USING TRANSFORMED T-CELLS**

(57) The present invention pertains to a method for culluring cord blood-derived natural killer cells using transformed T-cells. The method for culturing natural killer cells using transformed T-cells according to the present invention can effectively propagate and produce natural killer cells from a small amount of raw cells. In addition, the method can also improve the cell-killing ability of natural killer cells. Thus, the method for culturing natural killer cells using transformed T-cells according to the present invention can be usefully used to commercialize cell therapeutic agents. Moreover, natural killer cells produced by the culturing method of the present invention can be usefully used as a cell therapeutic agent.

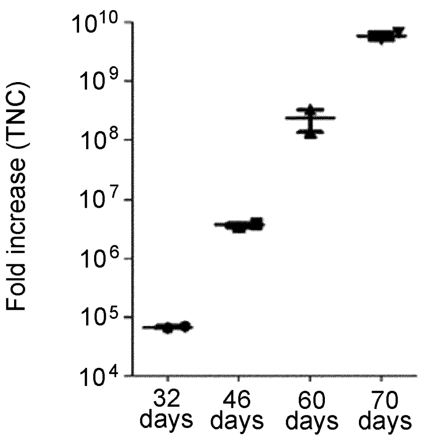


FIG. 2E



Printed by Jouve, 75001 PARIS (FR)

## Description

### Technical field

The present invention relates to a method for culturing cord blood-derived natural killer cells using transformed T-cells.

### Background art

Immunotherapy using the patient's immune function is being developed as a treatment for cancer patients and preventing recurrence. In particular, immunotherapy using natural killer cells capable of mass production and freezing is being studied. Natural killer cells are lymphocytic cells that account for about 15% of peripheral blood lymphocytes and play an important role in congenital immune responses.

Specifically, natural killer cells activate dendritic cells and induce cytotoxic T lymphocytes (CTL) to react specifically to tumors, thereby removing tumor cells. Natural killer cells directly kill malignant tumors, such as sarcoma, myeloma, carcinoma, lymphomas, and leukemia. However, most of the natural killer cells in the body of a normal person exist in an inactive state, and activated natural killer cells are required to remove the tumor. In addition, in the case of natural killer cells in the body of cancer patients, functional defects of natural killer cells exist due to the immune evasion mechanism of cancer cells.

Therefore, in order to use natural killer cells as a therapeutic agent, it is very important to activate natural killer cells. In addition, since the number of natural killer cells present in the body is limited, it is essential to develop a technology for proliferating and freezing the natural killer cells of the blood of a normal person or the blood of a patient in large quantities.

In vitro expansion method is used as a method for mass proliferation of natural killer cells, and a method for mass culture of natural killer cells using peripheral blood lymphocytes (PBMC), cord blood (CB), or human-induced pluripotent stem cells as raw materials is being studied.

In particular, unlike bone marrow, cord blood can be obtained through a simple procedure from cord blood that is discarded during parturition. In addition, since the industry for storing cord blood has been vitalized and it is also easy to find donors, studies are being actively carried out on a method for culturing natural killer cells using cord blood.

Specifically, methods for in vitro expansion culture of cord blood-derived natural killer cells include a method for proliferating using mononuclear cells (MNC) as seed cells and a method for proliferating using hematopoietic progenitor cells (CD34+ cells) as seed cells. The method using mononuclear cells as seed cells uses interleukin-2 (IL-2), interleukin-15 (IL-15), FLT-3L, etc. alone or in combination to help proliferate natural killer cells, but it has a problem of low proliferation rate and purity (Biossel L. et al., Biology of Blood and Marrow Transplantation, 14, 1031-1038, 2008). In addition, the method using hematopoietic progenitor cells as seed cells has a high proliferation rate and purity, but the culture period is long and various cytokines and growth factors must be used in combination, which presents difficulties in commercialization in terms of cost (Fias A.M. et al., Experimental Hematology 36(1):61-68, 2008).

PBMC, CD3- cells, CD3-CD56+ cells, CD56+ cells, etc. are used as seed cells for in vitro expansion culture of natural killer cells, and cytokines such as IL-2, IL-12, IL-15, and IL-21, LPS (Goodier et al., J. Immunol. 165(1):139-147, 2000), and OKT-3 antibody that stimulates CD3 (Condiotti et al., Experimental Hematol. 29(1):104-113, 2001) are used as natural killer cell proliferation factors. Said proliferation factors alone can proliferate natural killer cells by 3 to 10 times. However, it is difficult to commercialize natural killer cells as a therapeutic agent with the level of proliferation rate described above.

Recently, a method for mass proliferating natural killer cells using various types of feeder cells is being studied. Peripheral blood monocytes, EBV-LCL, and K562 cell lines are representative cell lines use as feeder cells. The K562 cell line is a blood cancer-derived cell line lacking HLA and is a representative target cell line that can be attacked easily by natural killer cells. For most of the feeder cells for culturing natural killer cells, a method for proliferating by expressing 4-1BBL and membrane-bound IL-15 in K562 cell line (Fujisaki et al., Cancer Res. 69(9):4010-4017, 2009), a method for proliferating by expressing MICA, 4-1BBL, and IL-15 (Gong et al., Tissue Antigens, 76(6):467-475, 2010), a method for proliferating by expressing 4-1BBL and membrane-bound IL-21, etc. are known.

### Detailed description of the invention

### Technical problem(s)

Accordingly, in order to efficiently proliferate natural killer cells from cord blood, the present inventors have co-cultured cord blood-derived natural killer cells and CD4+ T cells that have expressed co-stimulating factors and growth factors capable of increasing the proliferation of natural killer cells to develop a method for in vitro proliferation.

Specifically, in order to increase the efficiency of the method for culturing natural killer cells using the CD4(+) T cells as feeder cells, the present inventors produced transformed CD4(+) T cells. The present invention was completed by co-culturing the transformed CD4(+) T cells and cord blood-derived mononuclear cells and confirming that the proliferation rate and cell killing ability of natural killer cells are increased through such co-culture.

**Means to solve the problem(s)**

One aspect of the present invention provides a method for culturing natural killer cells comprising a step for co-culturing transformed CD4+ T cells and seed cells.

Another aspect of the present invention provides natural killer cells produced by said culture method.

**Effect of the invention**

The method for culturing natural killer cells using transformed T cells of the present invention can be produced by effectively proliferating natural killer cells from a small amount of cord blood-derived seed cells. In addition, the natural killer cells produced in this manner have improved cell killing ability. Therefore, the method for culturing natural killer cells using transformed T cells of the present invention can be usefully used for the commercialization of cell therapy agents. Furthermore, the natural killer cells produced by the culture method of the present invention can be usefully used as a cell therapy agent.

**Brief description of drawings**

FIG. 1 a is a diagram confirming the expression status of the gene in Hut78 cell line through FACS.

FIG. 1b is a diagram confirming the expression status of a single gene introduced into Hut78 cell line through FACS.

FIG. 1c is a diagram confirming the expression status of mTNF-$\alpha$/OX40L and mTNF-$\alpha$/4-1BBL dual genes introduced into Hut78 cell line through FACS.

FIG. 1d is a diagram confirming the expression status of mbIL-21/OX40L and mbIL-21/4-1BBL dual genes introduced into Hut78 cell line through FACS.

FIG. 1e is a diagram confirming the expression status of triple genes introduced into Hut78 cell line through FACS.

FIG. If is a diagram confirming the expression status of quadruple genes introduced into Hut78 cell line through FACS.

FIG. 2a is a diagram illustrating the proliferation rate of natural killer cells produced by co-culturing Hut78 cell line into which the gene has been introduced and cord blood-derived CD3(-) mononuclear cells for each transgene.

FIG. 2b is a diagram illustrating the proliferation rate of natural killer cells produced by co-culturing H9 cell line into which the gene has been introduced and cord blood-derived CD3(-) mononuclear cells for each transgene.

FIG. 2c is a diagram illustrating the proliferation rate of natural killer cells produced by co-culturing Jurkat cell line into which the gene has been introduced and cord blood-derived CD3(-) mononuclear cells for each transgene.

FIG. 2d is a diagram illustrating the proliferation rate of natural killer cells produced by co-culturing Peer cell line into which the gene has been introduced and cord blood-derived CD3(-) mononuclear cells for each transgene.

FIG. 2e is a diagram illustrating the proliferation rate of natural killer cells produced by restimulating at 14-day or 16-day interval when co-culturing Hut78 cell line into which the triple gene has been introduced and cord blood-derived CD3(-) mononuclear cells.

FIG. 3a is a diagram illustrating the survival rate of natural killer cells produced by co-culturing Hut78 cell line into which the gene has been introduced and cord blood-derived CD3(-) mononuclear cells for each transgene.

FIG. 3b is a diagram illustrating the survival rate of natural killer cells produced by co-culturing H9 cell line into which the gene has been introduced and cord blood-derived CD3(-) mononuclear cells for each transgene.

FIG. 3c is a diagram illustrating the survival rate of natural killer cells produced by co-culturing Jurkat cell line into which the gene has been introduced and cord blood-derived CD3(-) mononuclear cells for each transgene.

FIG. 3d is a diagram illustrating the survival rate of natural killer cells produced by co-culturing Peer cell line into which the gene has been introduced and cord blood-derived CD3(-) mononuclear cells for each transgene.

FIG. 3e is a diagram illustrating the survival rate of natural killer cells produced by restimulating at 14-day or 16-day interval when co-culturing Hut78 cell line into which the triple gene has been introduced and cord blood-derived CD3(-) mononuclear cells.

FIG. 4a is a diagram illustrating the purity (CD3-CD56+) of natural killer cells produced by co-culturing Hut78 cell line into which the gene has been introduced and cord blood-derived CD3(-) mononuclear cells for each transgene.

FIG. 4b is a diagram illustrating the purity (CD3-CD56+) of natural killer cells produced by co-culturing H9 cell line into which the gene has been introduced and cord blood-derived CD3(-) mononuclear cells for each transgene.

FIG. 4c is a diagram illustrating the purity (CD3-CD56+) of natural killer cells produced by co-culturing Jurkat cell line into which the gene has been introduced and cord blood-derived CD3(-) mononuclear cells for each transgene.

FIG. 4d is a diagram illustrating the purity (CD3-CD56+) of natural killer cells produced by co-culturing Peer cell line into which the gene has been introduced and cord blood-derived CD3(-) mononuclear cells for each transgene.

FIG. 4e is a diagram illustrating the purity (CD3-CD56+) of natural killer cells produced by restimulating at 14-day or 16-day interval when co-culturing Hut78 cell line into which the triple gene has been introduced and cord blood-derived CD3(-) mononuclear cells.

FIG. 5a is a diagram illustrating the activity (CD16+CD56+) of natural killer cells produced by co-culturing Hut78 cell line into which the gene has been introduced and cord blood-derived CD3(-) mononuclear cells for each transgene.

FIG. 5b is a diagram illustrating the expression level of the NKG2D phenotype marker of natural killer cells produced by co-culturing Hut78 cell line into which the gene has been introduced and cord blood-derived CD3(-) mononuclear cells by each transgene.

FIG. 5c is a diagram illustrating the expression level of the NKp30 phenotype marker of natural killer cells produced by co-culturing Hut78 cell line into which the gene has been introduced and cord blood-derived CD3(-) mononuclear cells by each transgene.

FIG. 5d is a diagram illustrating the expression level of the NKp44 phenotype marker of natural killer cells produced by co-culturing Hut78 cell line into which the gene has been introduced and cord blood-derived CD3(-) mononuclear cells by each transgene.

FIG. 5e is a diagram illustrating the expression level of the NKp46 phenotype marker of natural killer cells produced by co-culturing Hut78 cell line into which the gene has been introduced and cord blood-derived CD3(-) mononuclear cells by each transgene.

FIG. 5f is a diagram illustrating the expression level of the DNAM-1 phenotype marker of natural killer cells produced by co-culturing Hut78 cell line into which the gene has been introduced and cord blood-derived CD3(-) mononuclear cells by each transgene.

FIG. 5g is a diagram illustrating the expression level of the CXCR3 phenotype marker of natural killer cells produced by co-culturing Hut78 cell line into which the gene has been introduced and cord blood-derived CD3(-) mononuclear cells by each transgene.

FIG. 6a is a diagram illustrating the activity (CD16+CD56+) of natural killer cells produced by co-culturing H9 cell line into which the gene has been introduced and cord blood-derived CD3(-) mononuclear cells for each transgene.

FIG. 6b a diagram illustrating the expression level of the NKG2D phenotype marker of natural killer cells produced by co-culturing H9 cell line into which the gene has been introduced and cord blood-derived CD3(-) mononuclear cells by each transgene.

FIG. 6c is a diagram illustrating the expression level of the NKp30 phenotype marker of natural killer cells produced by co-culturing H9 cell line into which the gene has been introduced and cord blood-derived CD3(-) mononuclear cells by each transgene.

FIG. 6d is a diagram illustrating the expression level of the NKp44 phenotype marker of natural killer cells produced by co-culturing H9 cell line into which the gene has been introduced and cord blood-derived CD3(-) mononuclear cells by each transgene.

FIG. 6e is a diagram illustrating the expression level of the NKp46 phenotype marker of natural killer cells produced by co-culturing H9 cell line into which the gene has been introduced and cord blood-derived CD3(-) mononuclear cells by each transgene.

FIG. 6f is a diagram illustrating the expression level of the DNAM-1 phenotype marker of natural killer cells produced by co-culturing H9 cell line into which the gene has been introduced and cord blood-derived CD3(-) mononuclear cells by each transgene.

FIG. 6g is a diagram illustrating the expression level of the CXCR3 phenotype marker of natural killer cells produced by co-culturing H9 cell line into which the gene has been introduced and cord blood-derived CD3(-) mononuclear cells by each transgene.

FIG. 7a is a diagram illustrating the expression level of NKG2D phenotype marker and the activity (CD16+CD56+) of natural killer cells produced by restimulating at 14-day or 16-day intervals when co-culturing Hut78 cell line into which the triple gene has been introduced and cord blood-derived CD3(-) mononuclear cells.

FIG. 7b is a diagram illustrating the expression level of NKp30, NKp44, NKp46, DNAM-1, and CXCR3 phenotype markers of natural killer cells produced by restimulating at 14-day or 16-day interval when co-culturing Hut78 cell line into which the triple gene has been introduced and cord blood-derived CD3(-) mononuclear cells.

FIG. 8a is a diagram illustrating the tumor cell killing ability of natural killer cells produced by co-culturing Hut78 cell line into which the gene has been introduced and cord blood-derived CD3(-) mononuclear cells by each transgene.

FIG. 8b is a diagram illustrating the tumor cell killing ability of natural killer cells produced by co-culturing H9 cell line into which the gene has been introduced and cord blood-derived CD3(-) mononuclear cells by each transgene.

FIG. 8c is a diagram illustrating the tumor cell killing ability of natural killer cells produced by co-culturing Jurkat cell line into which the gene has been introduced and cord blood-derived CD3(-) mononuclear cells by each transgene.

FIG. 8d is a diagram illustrating the tumor cell killing ability of natural killer cells produced by co-culturing Peer cell line into which the gene has been introduced and cord blood-derived CD3(-) mononuclear cells by each transgene.

FIG. 8e is a diagram illustrating the tumor cell killing ability of natural killer cells produced by restimulating at 14-day or 16-day interval when co-culturing Hut78 cell line into which the triple gene has been introduced and cord blood-derived CD3(-) mononuclear cells.

FIG. 9a is a diagram illustrating an administration schedule for efficacy evaluation using the Raji mouse animal model.

FIG. 9b is a diagram illustrating the result of measuring the survival rate for confirming the efficacy of NK cells, RTX, and co-administration in the Raji animal model.

FIG. 10a is a diagram illustrating an administration schedule for efficacy evaluation using the Ramos mouse animal model.

FIG. 10b is a diagram illustrating the result of measuring the survival rate for confirming the efficacy of NK cells, RTX, and co-administration in the Ramos animal model.

## Best mode for carrying out the invention

Hereinafter, the present invention will be described in detail.

One aspect of the present invention provides a method for culturing natural killer cells comprising a step for co-culturing transformed CD4+ T cells and seed cells.

The transformed CD4+ T cells may express at least one gene selected from the group composed of 4-1BBL gene, mbIL-21 gene, OX40L gene, and mTNF-α gene.

Specifically, when one gene is introduced into the transformed CD4+ T cells, the gene may be 4-1BBL, mbIL-21, OX40L, or mTNF-α. In addition, when two genes are introduced into the transformed CD4+ T cells, said gene combination may be mbIL-21/4-1BBL, 4-1BBL/OX40L, mTNF-α/4-1BBL, mbIL-21/OX40L, mbIL-21/mTNF-α or mTNF-α/OX40L. In one embodiment of the present invention, genes of a combination of mbIL-21/4-1BBL, mTNF-α/OX40L, mTNF-α/4-1BBL and mbIL-21/OX40L were introduced into T cells.

In addition, when three genes are introduced into the transformed CD4+ T cells, said gene combination may be 4-1BBL/mbIL-21/OX40L, mbIL-21/OX40L/mTNF-α, mTNF-α/ mbIL-21 /4-1BBL or 4-1BBL/OX40L/mTNF-α. In one embodiment of the present invention, genes of a combination of mTNF-α/ mbIL-21 /4-1BBL were introduced into T cells.

In addition, when four genes are introduced into the transformed CD4+ T cells, said gene combination may be mTNF-α/mbIL-21/OX40L/4-1BBL. In one embodiment of the present invention, genes of a combination of mTNF-α/mbIL-21/OX40L/4-1BBL were introduced into T cells.

The term '4-1BBL' used in the present invention is one of TNFSF (TNF superfamily) called CD137L and refers to a ligand that binds to the receptor 4-1BB by forming a trimer. The 4-1BB gene may be derived from humans.

Specifically, the 4-1BBL gene may be NCBI Reference Sequence: NM_003811, but is not limited thereto. The 4-1BBL gene may be a base sequence coding the amino acid sequence represented by sequence No. 1. The base sequence coding the amino acid sequence represented by sequence No. 1 may be a base sequence represented by sequence No. 2.

The term 'mbIL-21' used in the present invention may be IL-21 designed to be bound to a cell membrane. Here, mbIL-21 may be a fusion protein in which IL-21 and a transmembrane protein are combined. The transmembrane protein may be CD8α. Specifically, it may be a transmembrane domain of CD8α.

Specifically, the IL-21 gene may be NCBI Reference Sequence: NM_021803.3, but is not limited thereto. In addition, the CD8α gene may be NCBI Reference Sequence: NM_001768, but is not limited thereto. The mbIL-21 is expressed in the form of IL-21 bound to the cell membrane. In addition, the mbIL-21 gene may be a base sequence coding the amino acid sequence represented by sequence No. 3. The base sequence coding the amino acid sequence represented by sequence No. 3 may be a base sequence represented by sequence No. 4.

The term 'OX40L' used in the present invention is also called TNFSF4, gp34, TXGP1, CD252, and CD134L, and refers to a ligand that binds to OX40. Specifically, the OX40L gene may be NCBI Reference Sequence: NM_003326, but is not limited thereto. The OX40L gene may be a base sequence coding the amino acid sequence represented by sequence No. 5. The base sequence coding the amino acid sequence represented by sequence No. 5 may be the base sequence represented by sequence No. 6.

The term 'mTNF-α' used in the present invention refers to the gene in which Alanine-Valine, which is a TACE (tumor necrosis factor-alpha-converting enzyme) recognition site, has undergone a point mutation in DNA in the amino acid sequence of tumor necrosis factor-alpha to become Proline-Valine. Mutating alanine to proline was randomly chosen.

Specifically, the mTNF-α gene may be a base sequence coding the amino acid sequence represented by sequence No. 8. The base sequence coding the amino acid sequence represented by sequence No. 8 may be the base sequence represented by sequence No. 9.

The 4-1BBL gene, mbIL-21 gene, OX40L gene, or mTNF-α gene may be introduced through a recombinant lentivirus, but is not limited thereto.

As a method for transducing the gene into a cell, a biochemical method, a physical method, or a virus mediated transduction method may be used. In addition, as a biochemical method, FuGene6 (Roche, USA), Lipofectamine (LipofectamineTM 2000, Invitrogen, USA), or ExGen 500 (MBI Fermentas International Inc. CANADA) may be used. In addition, a lipid mediated method using lipofectamine may be used.

The term 'vector' used in the present invention is an expression vector capable of expressing a target gene in cells into which the vector has been introduced, refers to a gene construct comprising essential control elements operably connected so that the gene insert introduced into the vector can be expressed.

In addition, as the expression vector comprising the gene, any expression vector that can be expressed in a CD4+ cell line can be used, and in a specific embodiment of the present invention, pCDH-CMV-MCS-EF1-Puro (SBI, CD510B-1) or pCDH-CMV-MCS-EF1-Neo (SBI, CD514B-1) lentiviral vector was used.

The lentivirus refers to a virus of the retrovirus family characterized by a long incubation period. Lentiviruses can carry genetic information into the DNA of host cells. It is one of the most effective methods of gene transfer vectors capable of replicating in non-dividing cells.

**[0034]** * The CD4+ T cells may be CD4+ T cells isolated in vitro, CD4+ T cells expanded and cultured in vitro, or CD4+ cell lines (T lymphoma cell lines). In addition, the CD4+ T cells may be accessory T cells, and may be hybridomas obtained by fusing CD4+ T cells and cancer cells. Specifically, the CD4+ T cells may be any one selected from the group composed of Hut78, H9, Jurkat, Loucy, Molt-3, Molt-13, Peer, RPMI8402 and TALL-01 cells. Preferably, it may be Hut78, H9, Jurkat or Peer cells.

The term 'feeder cell' used in the present invention refers to a cell that is also called a culture support cell and does not proliferate but has the metabolic activity to help the proliferation of target cells by producing various metabolites. The feeder cells may be transformed CD4+ T cells expressing at least one gene selected from the group composed of 4-1BBL gene, mbIL-21 gene, OX40L gene, and mTNF-$\alpha$ gene.

The T cells used as the feeder cells may be inactivated cells in which divisional proliferation is inhibited or cells that have not been inactivated, and preferably, safety can be ensured by inactivation. As a method for inactivation, a common method known in the relevant industry may be used, and for example, a method for irradiating gamma-ray may be used. When using T cells that have not been inactivated, since most are tumor cells, they can be killed during culture by activated natural killer cells.

The term "seed cell" used in the present invention refers to a cell capable of proliferating into natural killer cells through appropriate culture. Specifically, the seed cell may be cord blood-derived mononuclear cells, or cord blood-derived natural killer cells. This is not limited thereto, and preferably, the seed cells may be CD3(-) cells from which CD3(+) cells have been removed.

As for the method for culturing natural killer cells, they may be cultured by mixing the feeder cells and the seed cells with a ratio of at least 0.1. Specifically, the ratio of the feeder cells and the seed cells may be 0.1:1 to 50:1. More specifically, it may be 0.5:1 to 40:1. Even more specifically, it may be 1:1 to 30:1. Most specifically, it may be 2:1 to 20:1. As a specific example, the ratio of the feeder cell and the seed cell may be 2.5:1, but is not particularly limited thereto. The "ratio" refers to a ratio based on the number of cells.

In the method for culturing natural killer cells, the seed cells may be mixed once with the feeder cells and cultured for 5 to 60 days, or mixed with the feeders cells at least twice and cultured for at least 60 days. Preferably, the seed cells may be mixed once with the feeder cells and cultured for 14 to 21 days, but it is not limited thereto.

In the method for culturing natural killer cells, natural killer cells and T lymphoma cell lines are co-cultured in a conventional animal cell culture medium, such as AIM-V media, RPMI1640, CellGro SCGM, X-VIVO20, IMDM, and DMEM. When co-cultured, interleukins and antibodies that have low affinity to T cells and stimulate T cells may be added for culture, but it is not limited thereto.

The term 'antibody that has low affinity to T cells and stimulates T cells' used in the present invention refers to a protein that specifically reacts to the CD3 antigen, which is a group of molecules that meets with the T cell receptor (TCR) to form an antigen recognition complex. Compared to TCR, the CD3 molecule has a longer intracellular region and plays a role of transmitting antigen recognition signals into the cell.

Preferably, an antibody, which has a low affinity to T cells and stimulates T cells, that can be used in the present invention may be an anti-CD3 antibody. Specifically, the anti-CD3 antibody may be OKT-3, UCHT1, or HIT3a.

The term 'interleukin' (IL) used in the present invention refers to a group of cytokines, and refers to a proteinaceous biological active substance produced by immune cells, such as lymphocytes, monocytes, and macrophages. The interleukin may be IL-2, IL-15, IL-12, IL-18, or IL-21.

In an embodiment of the present invention, it was cultured by adding OKT-3 antibody and IL-2. The concentration of the OKT-3 antibody added may be 0.1 ng/m$\ell$ to 1,000 ng/m$\ell$. Preferably, the concentration of the OKT-3 antibody may be 10 ng/$\mu\ell$. The concentration of IL-2 may be 10 U/m$\ell$ to 2,000 U/m$\ell$. Preferably, the concentration of IL-2 may be 1,000 U/ml. In addition, it may be cultured by adding additional growth factors that support the proliferation of serum or plasma and lymphocytes. The type of serum or plasma to be added to the medium is not particularly limited, and commercially available serum or plasma derived from various animals may be used. Preferably, human-derived serum or plasma derived from the person themselves may be used.

The term 'culture' of the present invention refers to a method for growing cells in an environmental condition that has been appropriately artificially controlled. The method for culturing the transformed CD4+ T cells may be performed using a method well known in the relevant industry. Specifically, said culture may be carried out in a continuous manner in a batch process, a fed batch, or a repeated fed batch process.

In addition, precursors suitable for the culture medium may be used. The raw materials described above may be added in a batch, fed batch, or continuous manner to the culture during the cultivation process, but it is not particularly limited thereto. Basic compounds, such as sodium hydroxide, potassium hydroxide, and ammonia, or acidic compounds, such as phosphoric acid or sulfuric acid, can be used in an appropriate manner to adjust the pH of the culture.

The culture method using T cells as feeder cells selectively induces culture of natural killer cells in seed cells, and it can be cultured stably without differences depending on the donor when proliferating natural killer cells compared to when using the donor's PBMC feeder cells. In addition, in vitro culture of cord blood seed cells is difficult when the donor's MNC is used as feeder cells. Therefore, the culture method using T cells as feeder cells can efficiently and stably secure a large amount of therapeutic natural killer cell agents for treatment.

Another aspect of the present invention provides natural killer cells produced by said method for culturing natural killer cells.

Natural killer cells cultured according to said method for culturing natural killer cells can be frozen and the function of the cells does not get damaged even when they are thawed again. In addition, since the expression of an activating receptor, such as NKp46, is high, the killing ability and secretion of cytokines against tumor cell lines are increased, and therefore, an excellent anticancer effect can be expected. Therefore, it is possible to manufacture a cell therapy product effective for tumor treatment using a large amount of clinically applicable activated natural killer cells.

In addition, natural killer cells produced by the method for culturing natural killer cells may be comprised in an amount of 10 to 95wt% based on the total weight of the composition for preventing or treating infectious diseases included as an active ingredient. In addition, the composition for preventing or treating infectious diseases or of the present invention may further comprise at least one type of active ingredients exhibiting the same or similar function in addition to said active ingredient.

The pharmaceutical composition for preventing or treating infectious diseases may be prepared into a pharmaceutical composition by comprising at least one type of pharmaceutically acceptable carriers in addition to the active ingredient described above for administration.

The dosage of the pharmaceutical composition for preventing or treating infectious diseases may be adjusted according to various factors including type of disease, severity of disease, type and content of active ingredients and other ingredients comprised in the composition, type of formulation, patient's age, weight, general health condition, gender, and diet, administration time, administration route, secretion rate of the composition, duration of treatment, and concurrently used drugs. However, for a desirable effect, the dost of natural killer cells according to the present invention may be $0.01x10^7$ cells/kg to $1.0x10^9$ cells/kg, and may be $0.5x10^7$ cells/kg to $1.0x10^8$ cells/kg. In this case, the administration may be carried out once a day, or may be divided into several administrations.

In addition, the pharmaceutical composition for preventing or treating infectious diseases may be administered to an individual by various methods known in the relevant industry. The administration route may be appropriately selected by a PHOSITA in consideration of the method of administration, volume of body fluid, viscosity, etc.

Another aspect of the present invention provides a composition for culturing natural killer cells comprising transformed CD4+ T cells as an active ingredient. Since the CD4+ T cells used in the present invention and the genes introduced into said cells have already been described above, the corresponding descriptions will be omitted to avoid excessive duplication.

## Mode for carrying out the invention

Hereinafter, the present invention will be described in detail by embodiments. However, the following embodiments are intended only for illustrating the present invention, and the present invention is not limited to the following embodiments.

### Embodiment 1. Production of recombinant lentivirus

### Embodiment 1.1. Production of recombinant lentiviral vector

For the lentiviral vector, pCDH-CMV-MCS-EF1-Puro (SBI, CD510B-1) or pCDH-CMV-MCS-EF1-Neo (SBI, CD514B-1) was used. For genes, 4-1BBL (TNF superfamily member 9, TNFSF9), mbIL-21 (membrane bound IL-21), OX40L (TNF superfamily member 4(TNFSF4) transcript variant 1), and mTNF-α (membrane bound TNF alpha) were used as transgenes.

Specifically, a 4-1BBL gene expression vector (Origene, RC211160) was used for the 4-1BBL gene (sequence No. 2). For the mbIL-21 gene (sequence No. 4), a pcDNA3.1 vector (Genscript, US) into which the codon-optimized mbIL-21 gene sequence has been inserted was used. The OX40L gene (sequence No. 6) was requested to be synthesized by Bioneer.

For mTNF-α gene (sequence No. 9), RNA was extracted from peripheral blood mononuclear cell (PBMC), and then CDS was obtained by RT(Reverse transcriptase)-PCR. TNF-α is cut by TACE (tumor necrosis factor-alpha-converting enzyme) to be secreted, and A-V (Alanine-Valine), which is a TACE recognition site, has undergone a point mutation in DNA in the TNF-α amino acid sequence to become P-V (Proline-Valine), thereby maintaining the state of being attached to the cell membrane. The point mutation was performed by substituting guanine, the $226^{th}$ base, with cytosine, and adenine, the $228^{th}$ base, with guanine in the human mTNF-α gene represented by sequence No. 7.

Using primers suitable for each transgene, CDS (Coding Sequence) of the transgene was amplified through PCR (Table 1).

[Table 1]

| Gene | Primer | Sequence information (5'->3') | Sequence number |
|---|---|---|---|
| 4-IBB L | 4-1BBL Forward | TCTAGAGCTAGCGAATTCGCCACCATG GAATACGCCTCTGACGCTT | Sequence number 10 |
| | 4-1BBL Reverse | TTCGCGGCCGCGGATCCTTATTCCGACC TCGGTGAAGG | Sequence number 11 |
| mbIL-21 | mbIL-21 Forward | TAGAGCTAGCGAATTCGCCACCGCCAC CATGGCTCTGCCC | Sequence number 12 |
| | mhIL-21 Reverse | TCGCGGCCGCGGATCCTCAATACAGGG TGATGACC | Sequence number 13 |
| OX40 L | OX40L Forward | TAGAGCTAGCGAATTCGCCACCATGGA ACGGGTGCAAC | Sequence number 14 |
| | OX40L Reverse | TCGCGGCCGCGGATCCTCACAAGACAC AGAACTCCCC | Sequence number 15 |
| mTNF -α | mTNF-α Forward | TAGAGCTAGCGAATTCGCCACCGCCAC CATGGCTCTGCCC | Sequence number 16 |
| | mTNF-α Reverse | TCGCGGCCGCGGATCCTCACAGGGCAA TGATCCC | Sequence number 17 |

Table 1 shows the primers used in the experiment. The transgene and lentiviral vector were treated with EcoRI and BamHI restriction enzymes. Then, it was ligated using In-Fusion HD cloning kit (Clontech, 639649). The ligated lentiviral vector was transformed in DH5α soluble cells (competent cells) and cultured. Plasmid DNA was obtained from the transformed DH5α soluble cells using a plasmid mini-prep kit (MACHEREY-NAGEL/740422.50). A request for sequencing was made to an external company and it was confirmed that all plasmid DNA matches the DNA sequence. In addition, the desired transgene was inserted into cLV-CMV-MCS-IRES-Puro (puromycin) or cLV-CMV-MCS-IRES-Neo (neomycin), cLV-CMV-MCS-IRES-Bsd (blasticidin) by an outsourced manufacturer by the same method as the one described above.

**Embodiment 1.2. Production of concentrated lentivirus**

In order to produce recombinant lentivirus, the 293T cell line was inoculated into a 75T flask (Nunc, 156499) with $1.5 \times 10^6$ to $2 \times 10^6$ cells 2 days before transfection, and cultured in an incubator at a temperature condition of 5% $CO_2$, 37°C. When the cell saturation of the 293T cells reached about 80% to 90%, the medium was replaced with 6 mℓ OPTI-MEM (Gibco, 31985-088) and incubated for 30 minutes at a temperature of 37°C and under the condition of 5% $CO_2$. A DNA mixture and a lipofectamine (lipofectamine 2000, Life technologies, 11668500) mixture were prepared (Table 2).

[Table 2]

| Category | Ingredients |
|---|---|
| DNA mixture | 6 μg target DNA, 6 μg Gag, 6 μg REV, 3 μg VSVG, 1 mℓ OPTI-MEM |
| Lipofectamine mixture | 36 μℓ lipofectamine 2000, 1 mℓ OPTI-MEM |

Table 2 shows the DNA mixture and the lipofectamine (lipofectamine 2000, Life technologies, 11668500) mixture. Each of the components of the mixtures was mixed well using a vortexer and left at room temperature for 3 minutes. Then, the two mixtures were mixed and left at room temperature for at least 20 minutes. 2 mℓ of a mixed solution of DNA and lipofectamine was treated with 293T cells being cultured in 6 mℓ OPTI-MEM. After 4 hours, it was replaced with DMEM (Gibco, 11995073) medium to which 10%(v/v) FBS has been added, and was cultured at a temperature of 37°C for 48 hours under the condition of 5% $CO_2$. 8mℓ of the culture solution of 293T cells cultured for 48 hours was collected and filtered through a 0.45 μm filter (Millipore, SLHP033RS). The filtered culture solution was concentrated to 250 μℓ or less using an Amicon Ultra-15 Centrifugal Filter Unit with Ultracel-100 membrane (Merckmillipore, UFC910096). The concentrated virus was divided into an appropriate amount and stored at a temperature of -80°C.

**Embodiment 2. Production of transgenic T cells**

**Embodiment 2.1. Lentivirus infection**

$0.5x10^6$ cell lines being cultured, 1mℓ OPTI-MEM, 50μℓ lentivirus thawing solution, and 10 μg/mℓ polybrene (Santa Cruz, C2013) were mixed and placed in a 6-well plate (Nunc, 140675), and spinoculation was performed for 90 minutes under 1800×g and at a temperature of 32°C. Then, after culturing in an incubator under a temperature condition of 5% $CO_2$, 37°C, it was replaced with an existing culture medium and cultured for 48 hours.

Hut78 cell line (ATCC, TIB-161™) was cultured in IMDM (ATCC, 30-2005) medium containing 20%(v/v) FBS. During subculture, the cell concentration was maintained at $1.5x10^5$ cells/mℓ to $2.0x10^5$ cells/mℓ. H9 cell line (ATCC, HTB-176™) and Jurkat cell line (ATCC, TIB-152™) were cultured in RPMI1640 (ATCC, 30-2001) medium containing 10%(v/v) FBS. During subculture, cell concentrations were maintained at $1.0x10^5$ cells/mℓ to $1.5x10^5$ cells/mℓ and $0.5x10^5$ cells/mℓ to $1.0x10^5$ cells/mℓ, respectively. Peer cell line was cultured in RPMI1640 medium containing 20%(v/v) FBS. During subculture, the cell concentration was maintained at $3.0x10^5$ to $5.0x10^5$ cells/mℓ. The subculture of all cell lines was performed at intervals of 2 to 3 days. A 75T flask was used as the culture vessel, and the amount of medium was maintained between 15 mℓ to 20 mℓ.

Cell lines infected with the recombinant lentivirus were selected using antibiotics (Table 3).

[Table 3]

| | Transgenic combination | Vector used | Cell line | Antibiotic usage concentration |
|---|---|---|---|---|
| Single gene expression | mTNF-amIL-2 1 | pCDH(System Biosciences, SBI) | Hut78 | 0.5 μg/mℓ puromycin (Life technologies, A1113802) |
| | OX40L4-1BBL | pCDH(System Biosciences, SBI) | Hut78 | 1 mg/mℓ G148(Sigma Aldrich, A1720-5G) |
| Double gene expression | mTNF-a/OX40L mbIL-21/OX40L mTNF-a/4-1BB L | pCDH(System Biosciences, SBI) | Hut78 | 0.5 μg/mℓ puramycinl mg/ mℓ G418 |
| | mhIL-21/4-1BB L | cLV (Sirion) | Hut78H9 JurkatPe er | 6 μg/mℓ Blasticidin (Invitroge n, R210-01)1 mg/ mℓG418 |
| Triple gene expression | mTNF-a/mbIL-2 1/4-1BBL | cLV (Sirion) | Hut78H9 Jurkat | 0.5 μg/mℓ puromycin6 μg/ mℓ Blasticidin l mg/mℓ G418 |
| Quadruple gene expression | mTNF-a/mbIL-2 l/OX40L/ 4-1BB L | mTNF-a/mbIL-2 1/4-1BBL: cLVOx40L: pCDH | Hut78 | 0.5 μg/mℓ puromycin6 μg/ mℓ Blasiticidin l mg/mℓ G418 |

Table 3 above shows the antibiotics used in cell lines into which the gene was introduced.

**Embodiment 2.2. Confirmation of transgene expression**

In order to confirm the expression of the transgene through flow cytometry, the cell lines subcultured in embodiment 2.1. were collected and centrifuged at 1,200 rpm for 5 minutes. Then, the culture solution was removed by suction. FACS buffer was created by adding 2%(v/v) FBS to PBS. The number of cells was measured by diluting with 1 mℓ of FACS buffer, and it was diluted with FACS buffer to a concentration of $5x10^6$ cells/mℓ. 100 μℓ of diluted cell solution was added to each of 5 mℓ FACS tubes (Falcon, 352052). After staining with anti-human TNF-a(membrane)-PE (R&D systems, FAB210P), anti-human OX40L-PE (BD, 558184), anti-human 4-1BBL-PE (BD, 559446), anti-human IL-21-PE (eBioscience, 12-7219-42), 7-AAD (Beckman coulter, IM3630c), PE mouse IgG1 κ isotype control (BD Pharmingen, 555749), and PerCP-Cy5.5 mouse IgG1 κ isotype control (BD, 550795), the expression rate of each gene was analyzed using FACS equipment (FIGS. 1a to If).

In addition, in order to confirm the expression of the transgene through RT-qPCR (Real time qPCR), the cell lines subcultured in embodiment 2.1. were collected and centrifuged at 1,200 rpm for 5 minutes. Then, the culture solution was removed by suction. The number of cells was measured by diluting with PBS, and RNA was isolated and quantified for 1x$10^6$ cells using an RNA prep kit. In addition, cDNA was synthesized using a cDNA synthesis kit. RT-qPCR was performed using the synthesized cDNA. Primers used in RT-qPCR are as shown in Table 4 below.

[Table 4]

| | Primer | Sequence information (5'->3') | Sequence number |
|---|---|---|---|
| 4.1BBL | Forward primer | TCTGAGACAGGGCATGTTTG | Sequence number 18 |
| | Reverse primer | CCACCAGTTCTTTGGTGTCC | Sequence number 19 |
| mTNF-α | Forward primer | AACCTCCTCTCTGCCATCAA | Sequence number 20 |
| | Reverse primer | ATAGTCGGGCCGATTGATCT | Sequence number 21 |
| mbIL-21 | Forward primer | TGGAAACAATGAGCGAATCA | Sequence number 22 |
| | Reverse primer | AACCGCTCCAGGAACTCTTT | Sequence number 23 |
| hTOP1 | Forward primer | CCAGACGGAAGCTCGGAAAC | Sequence number 24 |
| | Reverse primer | GTCCAGGAGGCTCTATCTTGAA | Sequence number 25 |

Table 4 shows the primers used in the RT-qPCR experiment. The expression level of the transgenes in the cell lines is shown in Table 5 below.

[Table 5]

| Ct value | TOP1 | mTNF-α | mhIL-21 | 4.1BBL |
|---|---|---|---|---|
| H9 | 20,3 | 21.5 | n.d | n.d |
| H9-mbIL-21-4.1BBL | 20.0 | 22.2 | 19.5 | 19.4 |
| H9-mTNF-α-mbIL-21-4.1BBL | 19.9 | 18.2 | 18.1 | 18.2 |
| Jurkat | 20.1 | 30.7 | n.d | n.d |
| Jurkat-mbIL-21-4,1BBL | 27.4 | 37.0 | 36.4 | 34.1 |

(continued)

| Ct value | TOP1 | mTNF-α | mhIL-21 | 4.1BBL |
|---|---|---|---|---|
| Jurkat-mTNF-α-mbIL-21-4.1BBL | 20.4 | 19.8 | 19.2 | 19.8 |
| Peer | 21.4 | 26.2 | 34.2 | 34.9 |
| Peer-mbIL-21-4.1BBL | 26.8 | 33.8 | 29.0 | 25.6 |
| *n.d: not detected | | | | |

As shown in Table 5, it was confirmed that the expression level of the genes introduced into the cell lines was increased.

**Embodiment 3. Co-cultivation of CD3(-) PBMC and transgenic T cells**

**Embodiment 3.1. Preparation of cord blood-derived CD3(-) PBMC seed cells**

Cord blood for research was placed in a 50 mℓ tube and centrifuged for 10 minutes at 1,500 rpm. Plasma of the upper layer was removed and PBS (phosphate buffered saline, LONZA, 17-516Q) was added in a 1:1 ratio. Then, after separating cord blood mononuclear cells (MNC) through Ficoll (Ficoll-Paque Plus, GE Healthcare, 17-1440-03) density gradient centrifugation method, the number of cells was measured using the ADAM cell counter system (Nano Entek).

In order to obtain seed cells from which CD3(+) cells have been removed, $5x10^7$ cord blood mononuclear cells were moved to a new 50 mℓ tube, and then centrifuged at 1,200 rpm and a temperature of 4°C for 5 minutes. A MACS running buffer containing 2%(v/v) FBS and EDTA with a concentration of 2 mM in PBS was prepared. After the centrifugation, 400 μℓ of MACS running buffer and 100 μℓ of CD3 magnetic beads (Miltenyi biotech, 130-050-101) were added to the pellet and reacted at a temperature of 4°C for 20 minutes. After washing by adding 10 mℓ MACS running buffer, it was centrifuged at 13,500 rpm and a temperature of 4°C for 8 minutes and suspended in 0.5 mℓ of MACS running buffer.

Cells were separated by mounting a CS column (Miltenyi Biotech, 130-041-305) on VarioMACS (Miltenyi Biotech). Cells were recovered by washing the column until finally reaching 20 mℓ. The recovered cells were placed in a new 50 mℓ tube, centrifuged at 1,200 rpm and a temperature of 4°C for 5 minutes, and suspended in a frozen medium. The number of cells was measured using the ADAM cell counter system to freeze $5x10^6$ cells per vial in liquid nitrogen.

One vial of frozen CD3(-) cord blood mononuclear cells was thawed in a water bath at a temperature of 37°C and moved to a 50 mℓ tube, suspended in PBS containing 0.6%(v/v) ACD (Citrate-dextrose solution, Sigma-Aldrich, C3821), 0.2%(v/v) FBS (Fetal serum bovine), and 2 mM EDTA, and centrifuged at 1,500 rpm and a temperature of 4°C for 10 minutes. CD3(-) cord blood mononuclear cells were suspended in CellGro medium (Cellgenix, 20802-0500), and the number of cells was measured using the ADAM cell counter system. CD3(-) cord blood mononuclear cells were suspended in CellGro medium at a concentration of $1x10^6$ cells/mℓ.

**Embodiment 3.2. Co-cultivation of CD3(-) cord blood mononuclear cells and transgenic T cells**

The transgenic T cells prepared in embodiment 2 were recovered from the culture flask and centrifuged at 1,200 rpm and a temperature of 4°C for 5 minutes. Then, it was suspended in CellGro medium, and the number of cells was measured using the ADAM cell counter system. The transgenic T cells were suspended in CellGro medium at a concentration of $2.5x10^6$ cells/mℓ, and then prepared by inactivating it with irradiation at 20,000 cGy in a gamma-ray irradiator.

When culturing natural killer cells, 1,000 IU of IL-2 (Proleukin Injection, Novartis Korea) and 10 ng/mℓ of OKT-3 (eBioscience, 16-0037-85) were placed in a culture plastic plate. On day 0 of cultivation, 0.25 mℓ of each of CD3(-) cord blood mononuclear cells and transgenic T cells was added at a ratio of 1:2.5, 0.25mℓ of CellGro medium containing 2%(v/v) human plasma was added, and stationary culture was carried out for 4 days in an incubator at a temperature condition of 37°C.

On the fourth day of cultivation, the same amount of CellGro medium containing 1%(v/v) human plasma and 1,000 IU/mℓ of IL-2 was added, and then stationary culture was performed again. Then, the number of cells was measured at intervals of 2 to 3 days, and suspension culture was carried out until the 21$^{st}$ day while adding CellGro medium containing 1%(v/v) human plasma and 1,000 IU/mℓ of IL-2 to reach a concentration of $1x10^6$ cells/mℓ. Proliferated natural killer cells were obtained by performing suspension culture until the 21$^{st}$ day. In this case, if the Jurkat cell lines or the Peer cell lines were used as feeder cells, the suspension culture was performed until the 11$^{th}$ day. If genes were introduced into H9 and Hut78 and used as feeder cells, the suspension culture was performed until the 21$^{st}$ day.

The result of comparing the proliferation rate of cultured natural killer cells showed that, based on the total number of cells (Total nucleated cells, TNC), when co-cultured with the Hut78 cell lines to which the gene was not introduced, it proliferated 93 times. It was confirmed that the proliferation rate of natural killer cells was significantly increased when co-cultured with the Hut78 cell lines into which one or more genes (mTNF-α, mbIL-21, 4-1BBL) were introduced. In particular, when co-cultured with the Hut78 cell lines into which the gene of mbIL-21/4-1BBL was introduced, it proliferated 957 times. In addition, when co-cultured with the Hut78 cell lines into which mTNF-α/mbIL-21/4-1BBL was introduced, it proliferated 1,138 times (Table 6, FIG. 2a).

[Table 6]

| **HuT78** + Transgene | **Average** | **STDEV** |
|---|---|---|
| HuT78 parental | 92.7 | 90.4 |
| mTNF-α | 112.3 | 67.2 |
| mbIL-21 | 448.1 | 251.4 |
| OX40L | 50.5 | 30.7 |
| 4.1BBL | 274.9 | 189.6 |
| mTNF-α+OX40L | 204.5 | 123.2 |
| mTNF-α+4.1BBL | 389,1 | 352.1 |
| mbIL-21+OX40L | 372.0 | 189.2 |
| mbIL-21+4.1BBL | 957.0 | 537.4 |
| mTNF-α+mbIL21+4.1BBL | 1138.5 | 192.0 |
| mTNF-α+OX40L+mbIL21+4.1BBL | 823.1 | 330.0 |

In addition, when co-cultured with the H9 cell lines into which the gene was not introduced, it proliferated 13 times, but when co-cultured with the H9 cell lines into which mbIL-21/4-1BBL or mTNF-α/mbIL-21/4-1BBL was introduced, it proliferated 367 times and 979 times, respectively (Table 7 and FIG. 2b).

[Table 7]

| **H9** + Transgene | **Average** | **STDEV** |
|---|---|---|
| H9 parental | 12.6 | 4.3 |
| mbIL21+4.1BBL | 367.4 | 80. 1 |
| mTNF-α+mbIL21+4.1BBL | 978.8 | 287.7 |

When co-cultured with other cell lines, such as Jurkat cell lines or Peer cell lines, cultivation was possible until the 11$^{th}$ day of culture. A relatively high proliferation rate was displayed in cell lines into which the mbIL-21/4.1BBL gene was introduced or cell lines into which the mTNF- α/mbIL-21/4-1BBL gene was introduced (Table 8 and Table 9, FIG. 2c and FIG. 2b).

[Table 8]

| **Jurkat** + Transgene | **Average** (11-day culture) | **STDEV** |
|---|---|---|
| Jurkat Parental | 0.9 | 0.7 |
| mbIL21+4.1BBL | 36.3 | 4,8 |
| mTNF-α+mbIL21+4.1BBL | 43.6 | 6.6 |

[Table 9]

| **Peer** + Transgene | **Average** (11-day culture) | **STDEV** |
|---|---|---|
| Peer Parental | 1.6 | 0.7 |
| mbIL21+4.1BBL | 14.3 | 4.1 |

The results described above showed that it is possible to culture natural killer cells by culturing CD3(-) cells isolated from cord blood mononuclear cells for 21 days with feeder cells into which the gene was introduced, and exhibited a higher proliferation that the non-transduced feeder cells.

**Embodiment 3.3. Restimulation of natural killer cell culture using Hut78 cells into which the mTNF-α/mbIL-21/4-1BBL gene was introduced**

The transgenic T cells prepared in embodiment 2 were recovered from the culture flask and centrifuged for 5 minutes at 1,200 rpm and a temperature of 4°C. Then, it was suspended in CellGro medium, and the number of cells was measured using the ADAM cell counter system. After suspending the transgenic T cells in CellGro medium at a concentration of $2.5x10^6$ cells/mℓ, it was prepared by inactivating it with irradiation at 20,000 cGy in a gamma-ray irradiator.
When culturing natural killer cells, 1,000 IU of IL-2 and 10 ng/mℓ of OKT-3 were placed in a culture plastic plate. On day 0 of cultivation, 0.25 mℓ to 1 mℓ of each of CD3(-) cord blood mononuclear cells and transgenic T cells were added at a ratio of 1:2.5, 0.25 mℓ to 1 mℓ of CellGro medium containing 2%(v/v) human plasma was added, and stationary culture was carried out for 4 days in an incubator at a temperature condition of 37°C.
On the fourth day of cultivation, the same amount of CellGro medium containing 1%(v/v) human plasma and 1,000 IU/mℓ of IL-2 was added, and then stationary culture was performed again. Then, the number of cells was measured at intervals of 2 to 3 days, and cultivation was carried out while adding CellGro medium containing 1%(v/v) human plasma and 1,000 IU/mℓ of IL-2 to reach a concentration of $1x10^6$ cells/mℓ.
For restimulation, on day 0 of cultivation, HuT78 cells into which the mTNF-α/mbIL-21/4-1BBL was introduced were used at the same ratio. On the sixteenth day of cultivation, the first restimulation was given. First, the number of natural killer cells in cultivation was measured using the ADAM cell counter system, they were diluted with CellGro medium to become $1.5x10^6$ cells/mℓ, and 0.25 mℓ was prepared on a culture plastic plate. HuT78 cells into which the mTNF-α/mbIL-21/4-1BBL was introduced were suspended in CellGro medium to become $2.5x10^6$ cells/mℓ, and then prepared by inactivating it with irradiation at 10,000 cGy in a gamma-ray irradiator.
0.25 mℓ HuT78 cells into which the inactivated mTNF-α/mbIL-21/4-1BBL gene was introduced were added to a culture plastic. 1,000 IU/mℓ of IL-2 and 10 ng/mℓ of OKT-3, and 1%(v/v) human plasma were placed in a culture plastic plate, and stationary culture was carried out for 3 days in an incubator at a temperature of 37°C. Then, the number of cells was measured at intervals of 2 to 3 days, and cultivation was performed while adding CellGro medium containing 1%(v/v) human plasma and 1,000 IU/mℓ of IL-2 to reach a concentration of $1x10^6$ cells/mℓ. After the first restimulation, restimulation through feeder cells was performed on the $32^{nd}$, $46^{th}$, and $60^{th}$ day of culture in the name manner, and culture was continued until the $70^{th}$ day.
As a result, the proliferation rate of natural killer cells on the $32^{nd}$ day of cultivation after the first restimulation was $6.9x10^4$ times, $3.7x10^6$ times after the second restimulation, $2.3x10^8$ times on the $60^{th}$ day of cultivation after the third restimulation, and $5.9x10^9$ times on the $70^{th}$ day of cultivation after the fourth restimulation, maintaining sustained proliferation and showing a high proliferation rate (Table 10, FIG. 2e).

[Table 10]

| **Culturing day** | **Average** | **STDEV** |
|---|---|---|
| Day 32 | $6.9x10^4$ | $3.2x10^3$ |
| Day 46 | $3.7x10^6$ | $3.1x10^5$ |
| Day 60 | $2.3x10^8$ | $1.4x10^8$ |
| Day 70 | $5.9x10^9$ | $1.1x10^8$ |

Through this, it was confirmed that when a periodic restimulation was provided to HuT78 cell lines into which the mTNF-α/mbIL-21/4-1BBL was introduced, the proliferation rate continued to increase, making it an excellent feeder cell to be used.

**Experimental example 1. Confirmation of cell viability of natural killer cells according to transgenes**

In order to compare and evaluate the in-vitro cell viability, an ADAM cell counter system, which is one of the cell counters using PI staining solution capable of binding with the intracellular nucleus, was used. After calculating the number of viable cells by subtracting the number of dead cells from the measured total number of cells, cell viability was calculated using Equation I below.

[Equation I]

$$\text{Cell viability (\%)} = (\text{viable cell count} / \text{total cell count}) \times 100$$

In the case of natural killer cells co-cultured with HuT78 cell lines into which the gene was introduced, it exhibited viability of around 90% regardless of whether the gene was introduced (Table 11, FIG. 3a).

[Table 11]

| **HuT78** + Transgene | **Average** | **STDEV** |
|---|---|---|
| Parental | 91 | 2.6 |
| mTNF-α | 92.8 | 2.1 |
| mbIL-21 | 92.8 | 1.5 |
| QX40L | 90.3 | 1.3 |
| 4.1BBL | 91.3 | 1.3 |
| mTNF-α+OX40L | 93.5 | 1.7 |
| mTNF-α+4.1BBL | 92.5 | 1.7 |
| mbIL-21+OX40L | 89 | 2.4 |
| mhIL-21+4.1BBL | 89.8 | 2.6 |
| mTNF-α+mbIL-21+4.1BBL | 89 | 3.4 |
| QD | 88.5 | 3.4 |

In the case of other H9, Jurkat, or Peer cell lines, the viability of natural killer cells cultured in the cell line into which the mbIL-21/4-1BBL gene was introduced and the cell line into which the mTNF-α/mbIL-21/4-1BBL gene was introduced exhibited viability of at least 90% when cultured for 21 days (H9) and cultured for 11 days (Jurkat, Peer) (Tables 12 to 14,

FIGS. 3b to 3d).

[Table 12]

| **H9** + Transgene | **Average** | **STDEV** |
|---|---|---|
| Parental | 86 | 6.1 |
| mbIL21+4.1BBL | 91 | 3.1 |
| mTNF-α+mbIL21+4.1BBL | 94 | 0.6 |

[Table 13]

| **Jurkat** +Transgene | **Average** | **STDEV** |
|---|---|---|
| Parental | 80 | 6.1 |
| mhIL21+4.1BBL | 91 | 0.6 |
| mTNF-α+mbIL21+4.1BBL | 92 | 2.0 |

[Table 14]

| **Peer** + Transgene | **Average** | **STDEV** |
|---|---|---|
| Parental | 83.5 | 6.1 |
| mbIL21+4.1BBL | 91 | 0.6 |

In addition, as a result of culturing while increasing the number of restimulations with HuT78 into which the mTNF-α/mbIL-21/4-1BBL gene was introduced, the viability of natural killer cells shows high viability of about 90% or higher even when the number of restimulations was increased (Table 15, FIG. 3e).

[Table 15]

| Culturing day | Day 32 | Day 42 | Day 60 | Day 70 |
|---|---|---|---|---|
| Average | 96.0 | 93.5 | 97.5 | 91.5 |
| STDEV | 1.4 | 0.7 | 0.7 | 4.9 |

Through this, it was confirmed that since the natural killer cells maintain high viability even if the cultivation is continued for a long period of time, the expanded cultivation of natural killer cells is possible for a long period of time.

**Experimental example 2. Confirmation of purity of natural killer cells**

Natural killer cells cultured for 21 days or natural killer cells cultured by repeated restimulation were collected, centrifuged at 1,200 rpm for 5 minutes, and the culture solution was removed by suction. The number of cells was measured by diluting with 1 mℓ of FACS buffer, and was diluted with FACS butter to be 5x$10^6$ cells/mℓ. 100 μℓ of the diluted cell solution was added to each of 5 mℓ FACS tubes (Falcon, 352052), and the phenotype was analyzed with the following antibodies:

Tube 1: Anti-human CD3-FITC (BD Pharmingen, 555332), anti-human CD16-PE (BD Pharmingen, 555407), anti-human CD56-BV421 (BD Pharmingen, 562751)

Tube 2: Anti-human CD14-FITC (BD Pharmingen, 555397), anti-human CD19-PE (BD Pharmingen, 555413), anti-human CD3-BV421 (BD Pharmingen, 562438)

Tube 3: Anti-human CD3-FITC, anti-human NKG2D-PE (R&D system, FAB139P), anti-human CD56-BV421

Tube 4: Anti-human CD3-FITC, anti-humanNKp30-PE (BD Pharmingen, 558407), anti-human CD56-BV421

Tube 5: Anti-human CD3-FITC, anti-human NKp44-PE (BD Pharmingen, 558563), anti-human CD56-BV421

Tube 6: Anti-human CD3-FITC, anti-human NKp46-PE (BD Pharmingen, 557991), anti-human CD56-BV421

Tube 7: Anti-human CD3-FITC, anti-human DNAM-1-PE (BD Pharmingen, 559789), anti-human CD56-BV421

Tube 8: Anti-human CD3-FITC, anti-human CXCR3-PE (BD Pharmingen, 557185), anti-human CD56-BV421

Tube 9: Anti-human CD3-FITC, PE mouse IgGl κ isotype control (BD Pharmingen, 555749), anti-human CD56-BV421

Tube 10: FITC mouse IgGl κ isotype control (BD Pharmingen, 555748), PE mouse IgGl κ isotype control, BV421 mouse IgG1 κ isotype control (BD Pharmingen, 562438)

In tube 1 described above, the anti-human CD56 was carried out by selecting one of three fluorescence, and accordingly, the same fluorescence was selected for CD3 of tube 2, CD56 of tubes 3 to 9, and isotype control of tube 10.

The tubes were stained at refrigeration temperature for 30 minutes. Then, 2 mℓ of FACS buffer was added to the stained cells, and centrifuged at 1,500 rpm for 3 minutes. The supernatant was removed, 2 mℓ of FACS buffer was added again, and it was centrifuged at 2,000 rpm for 3 minutes. The supernatant was removed again, 200 μℓ of cytofix

buffer (fixation buffer, BD, 554655) was added and suspension was performed, and then FACS LSRII Fortessa (BD Biosciences) was used for confirmation of cells and investigation of purity and various phenotypes.

After co-culturing CD3(-) cells isolated from cord blood mononuclear cells with HuT78 cell lines into which the gene was introduced, natural killer cells were checked and purity was analyzed, and the result confirmed a high content of natural killer cells (CD3-CD56+) of 90% or higher in all conditions regardless of whether or not the gene was introduced (Table 16, FIG. 4a).

[Table 16]

| **HuT78** + Transgene | **Average** | **STDEV** |
|---|---|---|
| Parental | 92.4 | 9.6 |
| mTNF-α | 96.8 | 2.5 |
| mbIL-21 | 98.6 | 0.9 |
| OX40L | 95.7 | 3.6 |
| 4.1BBL | 98 | 1.8 |
| mTNF-α+OX40L | 97.2 | 2.5 |
| mTNF-α+4.1BBL | 98.6 | 1 |
| mbIL-21+OX40L | 98.4 | 1.3 |
| mbIL-21+4.1BBL | 98,5 | 0.9 |
| mTNF-α+mhIL-21+4.1BBL | 98.7 | 0.9 |
| QD | 99.3 | 0.5 |

In the case of other H9, Jurkat, or Peer cell lines, the natural killer cells co-cultured with the cell line into which the mbIL-21/4-1BBL gene or mTNF-α/mbIL-21/4-1BBL gene was introduced was confirmed and it was confirmed that its purity was maintained to be higher compared to the condition in which the gene is not introduced (Tables 17 to 19, FIGS. 4b to 4d).

[Table 17]

| **H9** + Transgene | **Average** | **STDEV** |
|---|---|---|
| Parental | 91.5 | 4.1 |
| mbIL21+4.1BBL | 98.5 | 0.7 |
| mTNF-α+mbIL21+4.1BBL | 99 | 0.3 |

[Table 18]

| **Jurkat** + Transgene | **Average** | **STDEV** |
|---|---|---|
| Parental | 88.6 | 6.9 |
| mbIL21+4.1BBL | 97.6 | 1.3 |
| mTNF-α+mbIL21+4.1BBL | 97.5 | 0.8 |

[Table 19]

| **Peer** +Transgene | **Average** | **STDEV** |
|---|---|---|
| Parental | 79.2 | 14.6 |
| mML21+4.1BBL | 94.9 | 2.1 |

In addition, for the natural killer cells cultured by increasing the number of restimulations with the cell line into which three genes, mTNF-$\alpha$/mbIL-21/4-1BBL, were introduced, a high content of natural killer cells (CD3-CD56+) of 90% or higher up to 60 days of cultivation was confirmed (Table 20, FIG. 4e).

[Table 20]

| Culturing day | Day 32 | Day 60 |
|---|---|---|
| Avergage | 99.7 | 97.8 |
| STDEV | 0.1 | 0,8 |

**Experimental example 3. Analysis of active markers of natural killer cells** In addition, after co-culturing CD3(-) cells isolated from cord blood mononuclear cells with feeder cells into which the gene was introduced for 21 days, receptor expression of representative natural killer cells was analyzed.

When co-cultured with HuT78 cell lines, all CD 16 was highly expressed, and all of them were highly expressed without any variation between donors under the condition of double transgenic feeder cells compared to the condition in which NKG2D, NKp30, NKp44, NKp46, and DNAM-1, which are active markers, were not introduced or the condition of single transgenic feeder cells (FIGS. 5a to 5g).

In addition, when co-cultured with H9 cell lines, it was confirmed that the expression levels of CD16 and NKG2D, DNAM-1, CXCR3 were higher when co-cultured with feeder cells into which the mbIL-21/4-1BBL gene and three genes, mTNF-$\alpha$/mbIL-21/4-1BBL, were introduced compared to the condition in which the gene was not introduced. The expression of other active markers, NKp30, NKp44, and NKp46, was highly expressed without variations between donors. Therefore, it was confirmed that the double and triple transgene feeder cells are useful feeder cells capable of increasing the activity of NK cells and tumor targeting (FIGS. 6a to 6g).

In addition, as a result of confirming the phenotype of co-cultured natural killer cells by restimulation using the Hut78 cell lines into which the three genes, mTNF-$\alpha$/mbIL-21/4-1BBL, were introduced, the expression of active markers, such as NKG2D, NKp44, NKp46, DNAM-1, and CXCR3, showed a tendency to decrease when cultured under the condition of being restimulated 4 times rather than 1 time. Through this, it was confirmed that as the number of restimulation increases, the culture period lengthens and may affect the expression level of some active markers (FIGS. 7a to 7b).

**Experimental example 4. Confirmation of cell killing ability of natural killer cells according to the transgene and co-culture of T cells**

$1x10^6$ K562 cancer cell lines were placed in a 15 mℓ tube and centrifuged. The cell pellet was suspended in RPMI1640 medium to which 1mℓ of 10%(v/v) FBS was added. Then, 30 $\mu$ℓ of 1 mM Calcein-AM (Molecular probe, C34852) was added, and then the light was blocked with foil, and it was stained for an hour in an incubator at a temperature condition of 37°C.

Tumor cell lines after Calcein-AM staining were washed by adding 10 mℓ to 15 mℓ of RPMI1640 medium to which 10%(v/v) FBS was added and centrifuged, and then the pellet was suspended in 10mℓ of RPMI1640 medium to which 10%(v/v) FBS was added to reach a concentration of $1x10^5$ cells/mℓ. For natural killer cells, $1x10^6$ cells were placed in a 15 mℓ tube and centrifuged, and the pellet was suspended in RPMI1640 medium to which 10%(v/v) FBS was added at the desired ratio (1:1) compared to the K562 cancer cell line. 100 $\mu$ℓ of each of the prepared K562 cancer cell line and the natural killer cell line were mixed and divided into a round-bottom 96-well plate (96-well U-bottom plate, Nunc, 163320), and each well was prepared in triplicate to obtain an average value.

100 $\mu$ℓ of the stained K562 cancer cell line was added to each Spon (Spontaneous release) well and 100 $\mu$ℓ of RPMI1640 medium to which 10%(v/v) FBS was added was inserted to each. 100 $\mu$ℓ of the stained K562 cancer cell lines was added to each Max (Maximum release) well and 100 $\mu$ℓ of triple distilled water to which 2%(v/v) Triton-X 100 was added was inserted to each.

In order to correct auto-fluorescence present in RPMI1640 medium to which 10%(v/v) FBS was added and RPMI1640 medium to which 2%(v/v) Triton-X 100 was added, a medium value was prepared by adding 200 $\mu$ℓ of RPMI1640 medium to which 10%(v/v) FBS was added, and 100 $\mu$ℓ of RPMI1640 medium to which 2%(v/v) Triton-X 100 was added was added to 100 $\mu$ℓ of RPMI1640 medium to which 10%(v/v) FBS was added to prepare the value of the mixture of the two solutions. The auto-fluorescence value was corrected by adding the difference (A) obtained by subtracting the value of the mixture from the medium value to the Max (Maximum release) value.

After blocking the light and reacting for 4 hours in an incubator at a temperature condition of 37°C, the plate was centrifuged at 2,000 rpm for 3 minutes. The supernatant was divided into 100 $\mu$ℓ on a 96-well black plate (Nunc, 237108). The fluorescence value ($OD_{480/535}$ nm) was measured using a fluorescent plate reader (Perkin Elmer, VICTOR X3), and the tumor cell killing ability of natural killer cells was calculated using Equation II below.

[Equation II]

% of killing = (Sample well average fluorescence value – Spon well average fluorescence value) / {(Max well average fluorescence value + A) – Spon well average fluorescence value} x 100

Natural killer cells cultured with various feeder cells were reacted with K562 cancer cell lines to measure the direct cell killing ability. As a result, for all feeder cells, the cell killing ability of natural killer cells cultured under the conditions in which the mbIL-21/4-1BBL gene and the mTNF-$\alpha$/mbIL-21/4-1BBL gene were introduced was increased compared to the condition in which the gene was not introduced (FIGS. 8a to 8d).

The cell killing ability of natural killer cells according to the number of restimulations of HuT78 cell lines into which the mTNF-$\alpha$/mbIL-21/4-1BBL gene was introduced exhibited a high killing ability up to 60 days of culture without significant difference (FIG. 8e).

Through this, it was confirmed that compared to feeder cells without genes introduced, feeder cells into which the mbIL-21/4-1BBL gene or mTNF-$\alpha$/mbIL-21/4-1BBL gene was introduced can be used usefully for in vitro expansion culture of high-purity natural killer cells having high activity as well as excellent cell killing ability.

**Embodiment 4. Animal experiment**

**Embodiment 4.1. Culture of natural killer cells using transgenic T feeder cells**

When culturing natural killer cells, 500 or 1000 IU/mL of IL-2 (2 (Proleukin Injection, Novartis Korea) and 10 ng/mL of OKT-3 (eBioscience, 16-0037-85) were placed in a culture plastic plate, and on day 0 of cultivation, CD3(-) cord blood mononuclear cells or peripheral blood mononuclear cells and transgenic T cells were added at a ratio of 1:2.5, CellGro medium containing 2%(v/v) human plasma was added, and stationary culture was carried out for 4 days in an incubator at 37°C. 1000 IU/mL of IL-2 was used for cord blood mononuclear cells and 500 IU/mL for peripheral blood mononuclear cells.

Thereafter, the cultivation of cord blood-derived natural killer cells was carried out by the following procedure: On the 4$^{th}$ day of cultivation, after adding the same amount of CellGro medium containing 1 v/v% human plasma and 1000 IU/mL of IL-2, stationary culture was carried out again. Then, the number of cells was measured at intervals of 2 to 3 days, CellGro medium containing 1 V/V% human plasma and 1000 IU/mL of IL-2 was added to reach 1 x $10^6$ cells/mL, and it was cultured until the 14$^{th}$ day. On the 14$^{th}$ day of cultivation, transgenic T feeder cells were restimulated at a ratio of 1:2.5 and cultured in CellGro medium containing 1 V/V% human plasma, OKT3, and IL-2. Then, the number of cells was measured at intervals of 2 to 3 days, CellGro medium containing 1 V/V% human plasma and 1000 IU/mL of IL-2 was added to reach 1 x $10^6$ cells/mL, and it was additionally cultured for 14 days, culturing cells for a total of 28 days.

Thereafter, the cultivation of peripheral blood-derived natural killer cells is as follows: On the 4$^{th}$ day of cultivation, after adding the same amount of CellGro medium containing 1 V/V% human plasma and 500 IU/mL of IL-2, stationary culture was carried out again. Then, the number of cells was measured at intervals of 2 to 3 days, CellGro medium containing 1 V/V% human plasma and 500 IU/mL of IL-2 was added to reach 1 x $10^6$ cells/mL, and it was cultured until the 11$^{th}$ day. On the 11$^{th}$ day of cultivation, transgenic T feeder cells were restimulated at a ratio of 1:2.5 and cultured in CellGro medium containing 1 V/V% human plasma, OKT3, and IL-2. Then, the number of cells was measured at intervals of 2 to 3 days, CellGro medium containing 1 V/V% human plasma and 1000 IU/mL of IL-2 was added to reach 1 x $10^6$ cells/mL, and it was additionally cultured for 8 to 10 days, culturing cells for a total of 19 to 21 days.

The cultured cells are suspended in a freezing medium to reach 1 x $10^6$ cells/mL, frozen using a temperature-controlled cell freezer, and stored in liquid nitrogen.

As a result of comparing the proliferation rate of cultured natural killer cells, CB-enFeeder proliferated approximately 80,000 times and PBMC-enFeeder proliferated 50,000 times, showing no significant difference (Table 21).

[Table 21]

| | Average | STDEV |
|---|---|---|
| CB-enFeeder | 79288.3 | 37381.0 |
| PBMC-en Feeder | 53649.3 | 16827.9 |

**Embodiment 4.2. Efficacy evaluation of the Raji animal model**

For the Raji-luci cell lines, cancer cells were collected on the last day of culture, the cell concentration was adjusted to 5x $10^5$ cells/mL using PBS, and then 0.2 mL (1 x $10^5$ cells/mouse) per mouse was injected into the tail vein. Natural killer cells were injected into the tail vein at 2 x $10^7$ cells/200$\mu$L, and Rituxan (hereinafter RTX, Mabthera Injection, Roche Korea) was diluted to a concentration of 0.01 $\mu$g/100$\mu$L using PBS and 100$\mu$L was injected under the skin of the weakened area between the scapular region and the chest wall of the mouse. NK cells were administered a total of 6 times to the tail vein using a fixator the day after cancer cell transplantation, and RTX was administered once under the skin (Table 22, FIG. 9a).

[Table 22]

| Group | Average | Administration | Volume ($\mu$l) | Number of animals |
|---|---|---|---|---|
| 1 | Frozen culture medium +IgG 0.01 $\mu$g /head | i.v+s.c | 200+100 | 10 |
| 2 | RTX 0.01 $\mu$g /head | s.c | 100 | 10 |
| 3 | PBMC-enFeeder NK 2x $10^7$cells/head | i.v | 200 | 10 |
| 4 | CB-eFeeder NK 2x $10^7$cells/head | i.v | 200 | 10 |
| 5 | PB-eFeeder NK +RTX | i.v+s.c | 200+100 | 10 |
| 6 | CB-eFeeder NK +RTX | i.v+s.c | 200+100 | 10 |

The observation of all animals was carried out twice a day and general symptoms and dead animals were observed, and after observing the death status, the median survival time of the frozen medium control group, natural killer cells, and RTX-treated group was calculated to evaluate the effect of prolonging the viability. After transplantation of the Raji-luci cell line, dead animals were observed over 26 to 122 days in two types of groups, natural killer cells and RTX alone and co-administration, and the median survival time until the last day (day 122) was 48.5 days, 43 days, and 47 days in the group administered with RTX, PBMC-enFeeder, and CB-enFeeder alone compared to 30 days for the frozen medium control group. The median survival time in the PBMC-enFeeder + RTX and CB-enFeeder + RTX co-administration group was shown to be at least 55 days and 75.5 days (Table 23, FIG. 9b).

[Table 23]

| Raji-luci model | Frozen culture medium | RTX | PBMC-en Feeder | CB-enFee der | RTX+PBMC-enFee der | RTX +CB-enFeed er |
|---|---|---|---|---|---|---|
| Average survival rate | 29.5 | 48.5 | 43 | 47 | 55 | 75.5 |

**Embodiment 4.3. Efficacy evaluation of the Ramos animal model**

For the Ramos cell lines, cancer cells were collected on the last day of cultivation, the cell concentration was adjusted to 5 x $10^6$ cells/mL using PBS, and then 0.2 mL (1 x $10^6$ cells/mouse) per mouse was injected into the tail vein. Natural killer cells were injected into the tail vein at 2 x $10^7$ cells/200$\mu$L, and RTX was diluted to a concentration of 0.03$\mu$g/ 100$\mu$L using PBS and 100$\mu$L was injected under the skin of the weakened area between the scapular region and the chest wall of the mouse. Natural killer cells were administered a total of 6 times to the tail vein using a fixator from the fourth day of cancer cell transplantation, and RTX was administered 6 times to the tail vein from the third day of cancer cell transplantation (Table 24, FIG. 10a).

[Table 24]

| Group | Amount | Administration | Volume ($\mu$l) | Number of animals |
|---|---|---|---|---|
| 1 | Frozen culture medium +IgG 0.3 $\mu$g /head | i.v+i.v | 200+100 | 10 |
| 2 | RTX 0.3 $\mu$g /head | i.v | 100 | 10 |
| 3 | PBMC-enFeeder NK 2x$10^7$cells/head | i.v | 200 | 10 |

(continued)

| Group | Amount | Administration | Volume (μl) | Number of animals |
|---|---|---|---|---|
| 4 | CB-eFeeder NK $2x10^7$cells/head | i.v | 200 | 10 |
| 5 | PB-eFeeder NK+RTX | i.v+i.v | 200+100 | 10 |
| 6 | CB-eFeeder NK +RTX | i.v+i.v | 200+100 | 10 |

The observation of all animals was carried out twice a day and general symptoms and dead animals were observed, and after observing the death status, the median survival time of the frozen medium control group, natural killer cells, and RTX-treated group was calculated to evaluate the effect of prolonging the viability. After transplantation of the Ramos cell line, dead animals were observed over 34 to 110 days in two types of groups, natural killer cells and RTX alone and co-administration, and the median survival time until the last day (day 124) was 49.5 days, 42 days, and 42.5 days in the group administered with RTX, PBMC-enFeeder, and CB-enFeeder alone compared to 31 days for the frozen medium control group. The median survival time in the PBMC-enFeeder + RTX and CB-enFeeder + RTX co-administration group was shown to be at least 63.5 days and 87.5 days (Table 25, FIG. 10b).

[Table 25]

| Ramos model | Frozen culture medium | RTX | PBMC-en Feeder | CB-enFeeder | RTX +PBMC-enFeeder | RTX + CB-en Feeder |
|---|---|---|---|---|---|---|
| Average survival rate | 31 | 49.5 | 42 | 42.5 | 63.5 | 87.5 |

As described above, specific parts of the present invention have been described in detail, and it is obvious to a PHOSITA that these specific technologies are only preferred embodiments, and the scope of the present invention is not limited thereto. Accordingly, it will be considered that the substantial scope of the present invention is defined by the appended claims and their equivalents.

## Claims

**1.** A method for culturing natural killer cells comprising a step for co-culturing transformed CD4+ T cells and seed cells.

**2.** The method for culturing natural killer cells of claim 1, wherein the transformed CD4+ T cells express at least one gene selected from the group composed of 4-1BBL gene, mbIL-21 gene, OX40L gene, and mTNF-$\alpha$ gene.

**3.** The method for culturing natural killer cells of claim 2, wherein the 4-1BBL gene is a base sequence coding the amino acid sequence represented by sequence No. 1.

**4.** The method for culturing natural killer cells of claim 3, wherein the base sequence coding the amino acid sequence represented by sequence No. 1 is a base sequence represented by sequence No. 2.

**5.** The method for culturing natural killer cells of claim 2, wherein the mbIL-21 gene is a base sequence coding the amino acid sequence represented by sequence No. 3.

**6.** The method for culturing natural killer cells of claim 5, wherein the base sequence coding the amino acid sequence represented by sequence No. 3 is a base sequence represented by sequence No. 4.

**7.** The method for culturing natural killer cells of claim 2, wherein the OX40L gene is a base sequence coding the amino acid sequence represented by sequence No. 5.

8. The method for culturing natural killer cells of claim 7, wherein the base sequence coding the amino acid sequence represented by sequence No. 5 is a base sequence represented by sequence No. 6.

9. The method for culturing natural killer cells of claim 2, wherein the mTNF-α gene is a base sequence coding the amino acid sequence represented by sequence No. 8.

10. The method for culturing natural killer cells of claim 9, wherein the base sequence coding the amino acid sequence represented by sequence No. 8 is a base sequence represented by sequence No. 9.

11. The method for culturing natural killer cells of claim 2, wherein the gene is introduced through a recombinant lentivirus.

12. The method for culturing natural killer cells of claim 1, wherein the CD4+ T cells express 4-1BBL gene or mbIL-21 gene.

13. The method for culturing natural killer cells of claim 1, wherein the CD4+ T cells express 4-1BBL gene and mbIL-21 gene.

14. The method for culturing natural killer cells of claim 1, wherein the CD4+ T cells express 4-1BBL gene, mbIL-21 gene, and mTNF-α gene.

15. The method for culturing natural killer cells of claim 1, wherein the CD4+ T cells express 4-1BBL gene, mbIL-21 gene, OX40L gene, and mTNF-α gene.

16. The method for culturing natural killer cells of claim 1, wherein the CD4+ T cells are any one selected group the group composed of Hut78, H9, Jurkat, Loucy, Molt-3, Molt-13, Peer, RPMI8402, and TALL-01 cells.

17. The method for culturing natural killer cells of claim 1, wherein the seed cells are mononuclear cells derived from cord blood.

18. The method for culturing natural killer cells of claim 1, wherein the seed cell is the cell from which CD3(+) cells have been removed.

19. The method for culturing natural killer cells of claim 1, wherein the cultivation is performed by mixing the transformed CD4+ T cells and seed sells at a ratio of 0.1:1 to 50:1.

20. The method for culturing natural killer cells of claim 1, wherein the seed cells are mixed once with the support cells and cultured for 5 to 60 days, or mixed with the support cells at least twice and cultured for at least 60 days.

21. The method for culturing natural killer cells of claim 1, wherein the cultivation is performed in a medium containing an anti-CD3 antibody and interleukin protein.

22. The method for culturing natural killer cells of claim 21, wherein the anti-CD3 antibody comprises any one selected from the group composed of OKT3, UCHT1, and HIT3a.

23. The method for culturing natural killer cells of claim 21, wherein the interleukin protein comprises any one selected from the group composed of IL-2, IL-12, IL-15, IL-18, and IL-21.

24. A natural killer cell produced by the method of culturing natural killer cells of claim 1.

25. A composition for culturing natural killer cells comprising transformed CD4+ T cells as an active ingredient.

26. The method for culturing natural killer cells of claim 25, wherein the transformed CD4+ T cells express at least one gene selected from the group composed of 4-1BBL gene, mbIL-21 gene, OX40L gene, and mTNF-α gene.

Hut78 parental

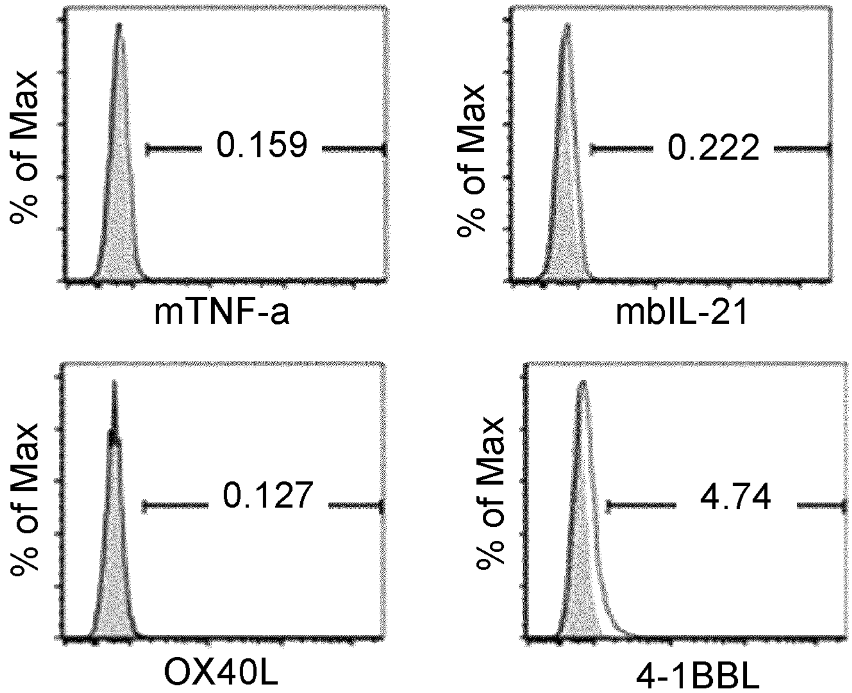


FIG. 1A

Hut78 feeder cells Single gene transduction

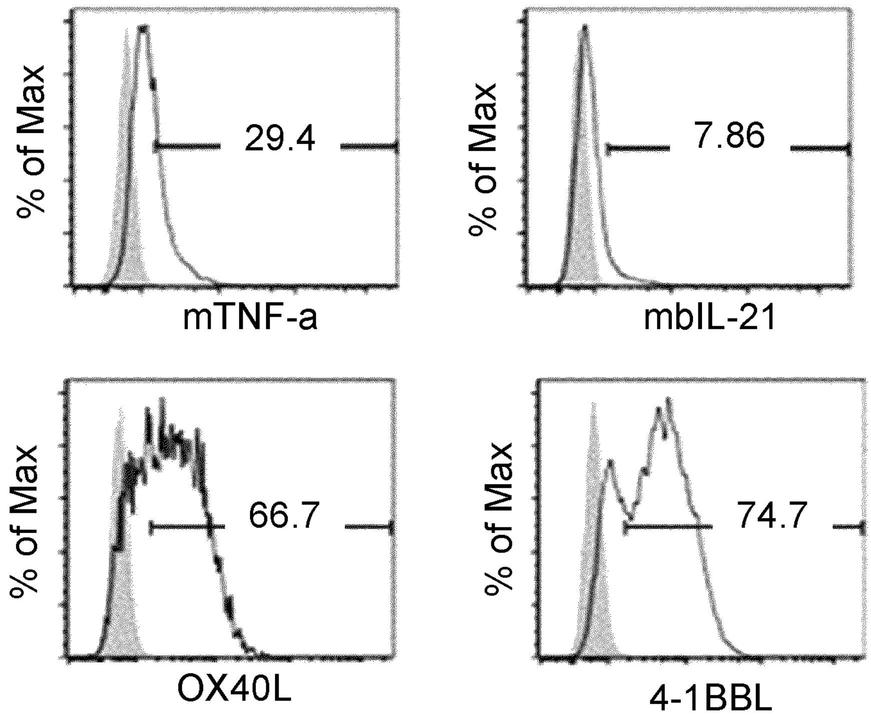


FIG. 1B

**Hut78 feeder cells**
**Double gene transduction**

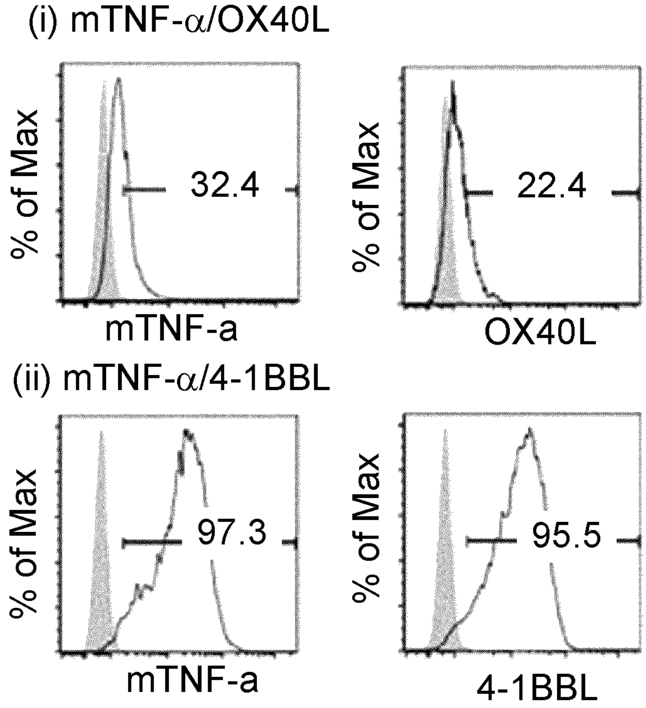


FIG. 1C

**Hut78 feeder cells**
**Double gene transduction**

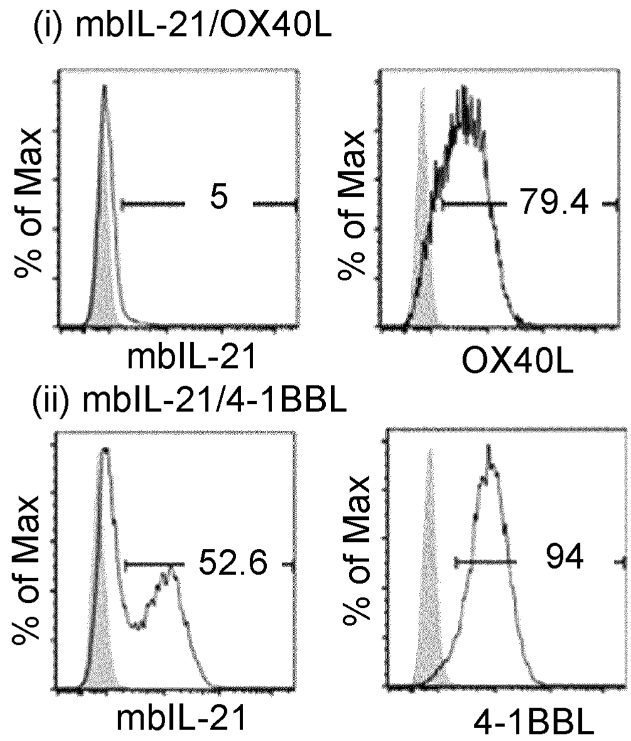


FIG. 1D

**Hut78 feeder cells**
**Triple gene transduction**

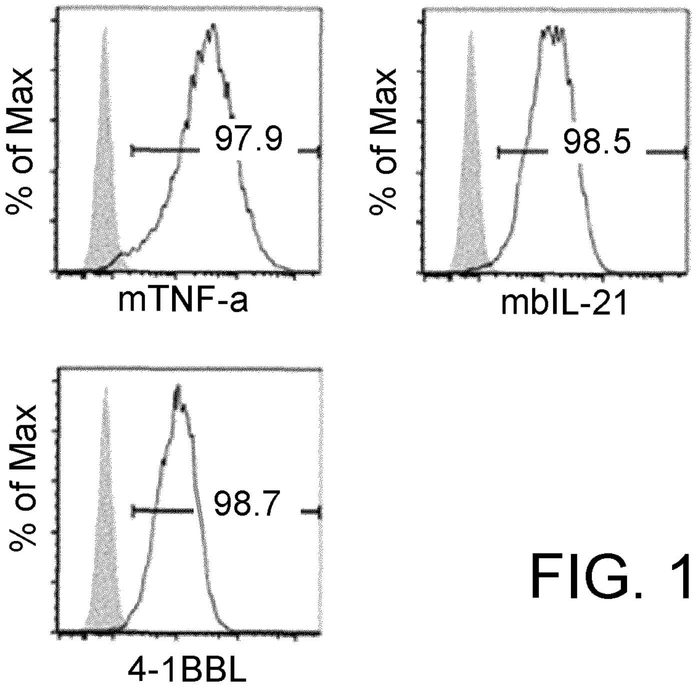


FIG. 1E

**Hut78 feeder cells**
**Quadruple gene transduction**

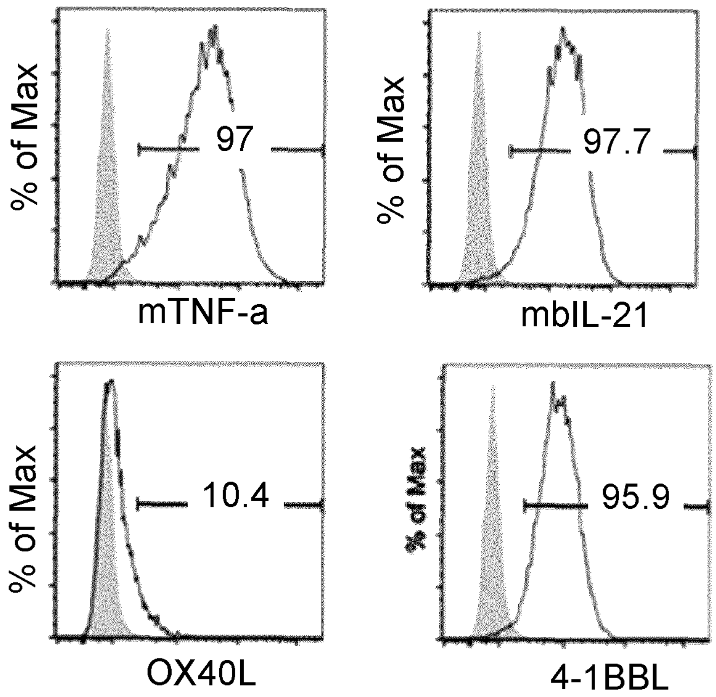


FIG. 1F

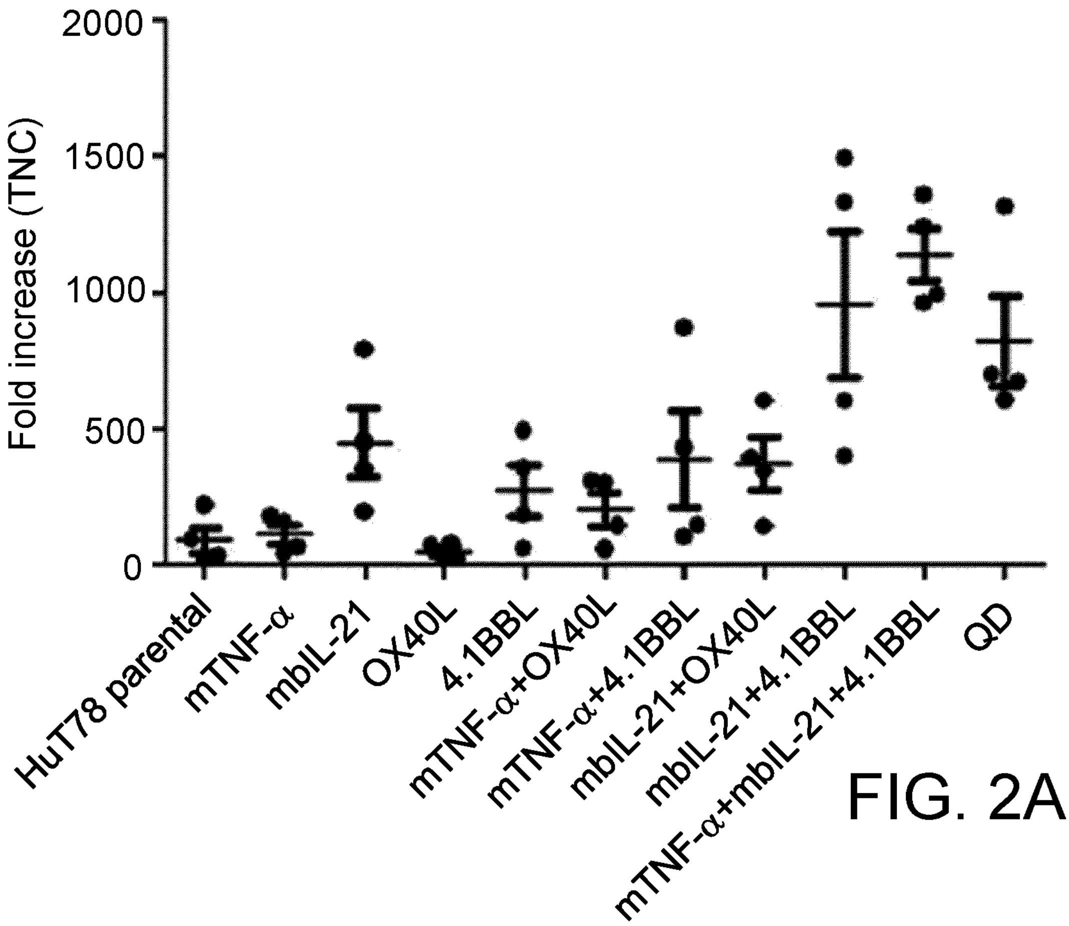

2000
1500
1000
500
0
Fold increase (TNC)
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD


FIG. 2A

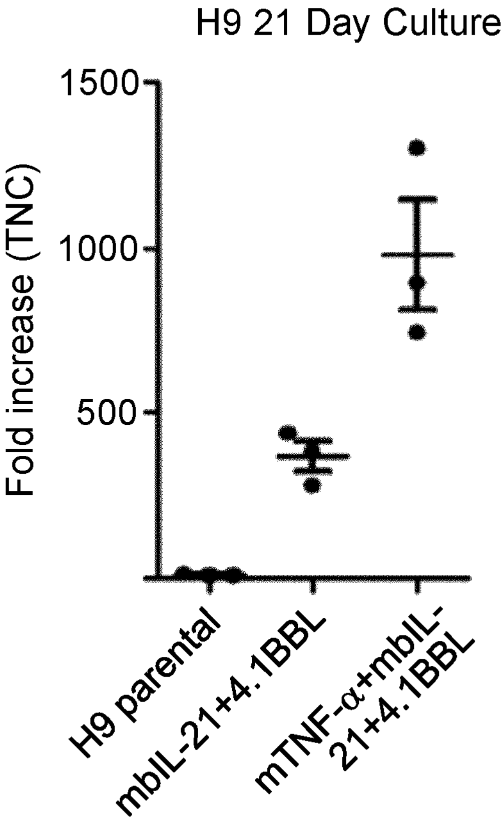

H9 21 Day Culture
1500
1000
500
Fold increase (TNC)
H9 parental
mbIL-21+4.1BBL
mTNF-α+mbIL-21+4.1BBL


FIG. 2B

Jurkat 11 Day Culture

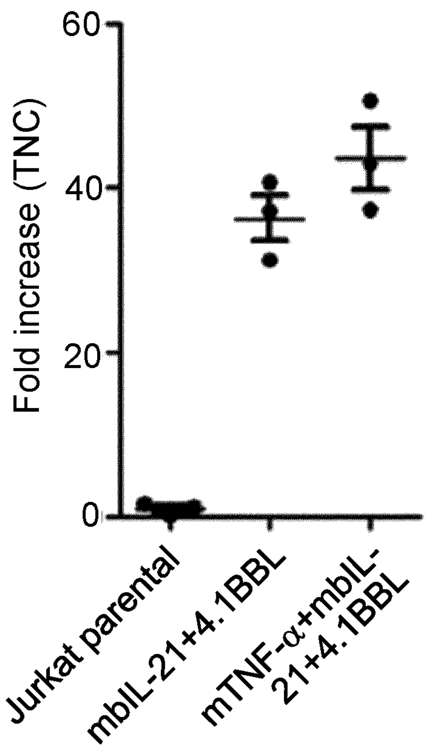


FIG. 2C

Peer 11 Day Culture

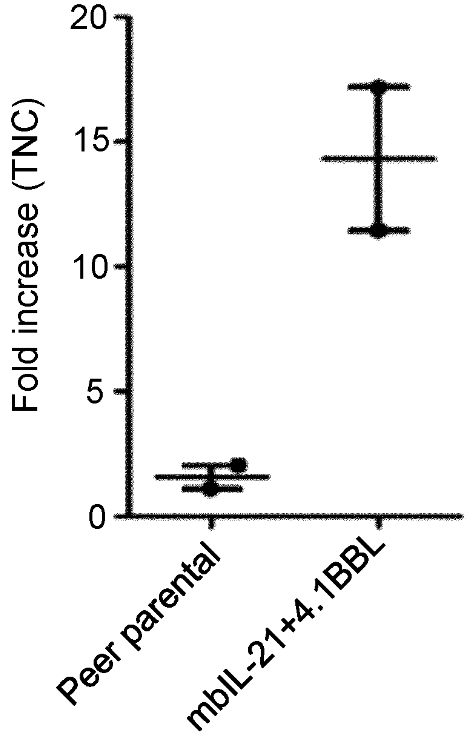


FIG. 2D

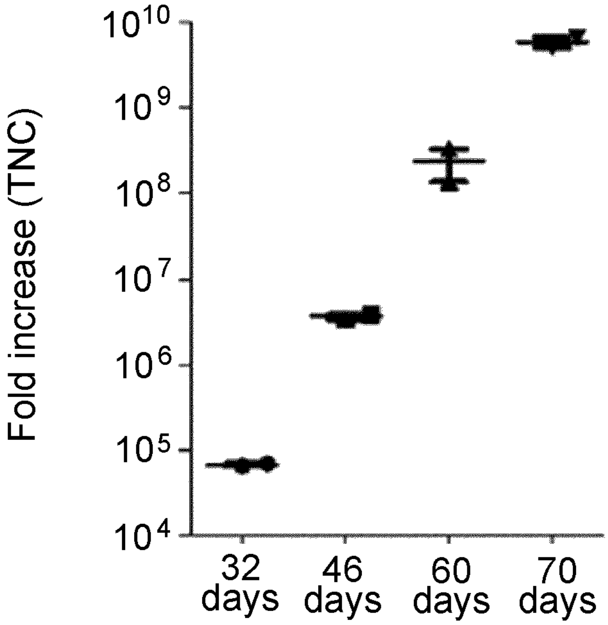
Fold increase (TNC)
10^10
10^9
10^8
10^7
10^6
10^5
10^4
32 days
46 days
60 days
70 days

FIG. 2E

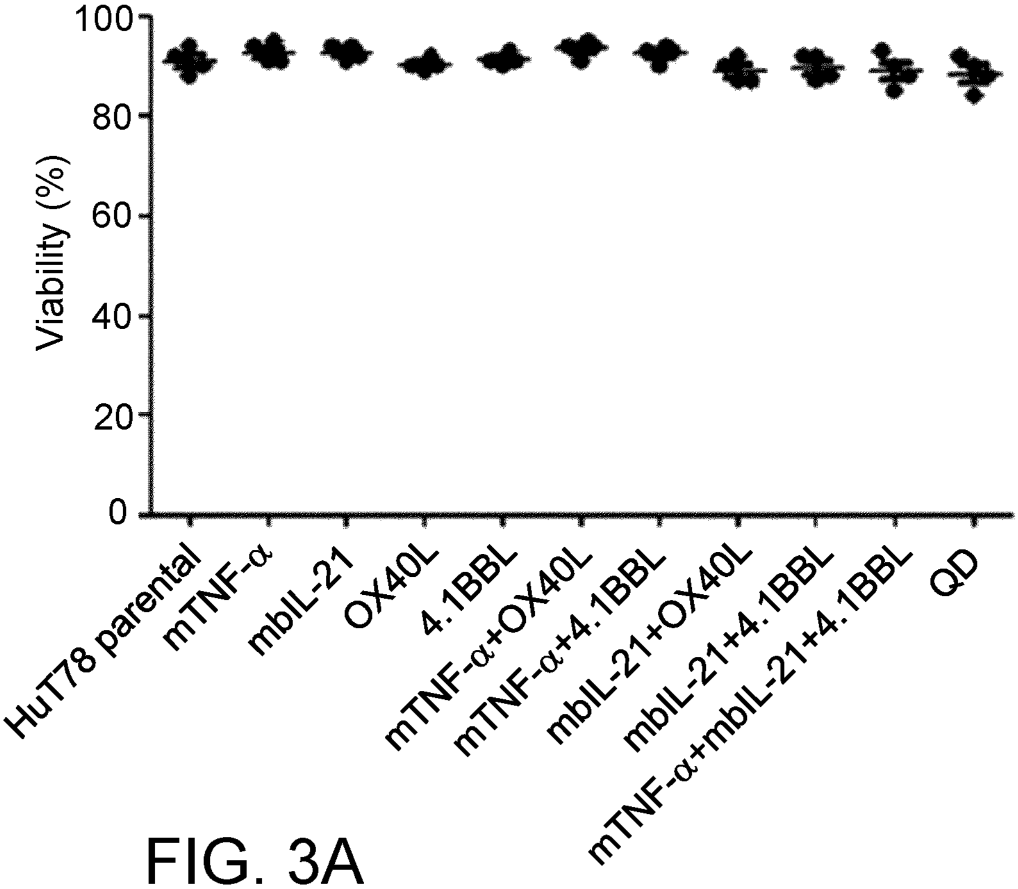
Viability (%)
100
80
60
40
20
0
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 3A

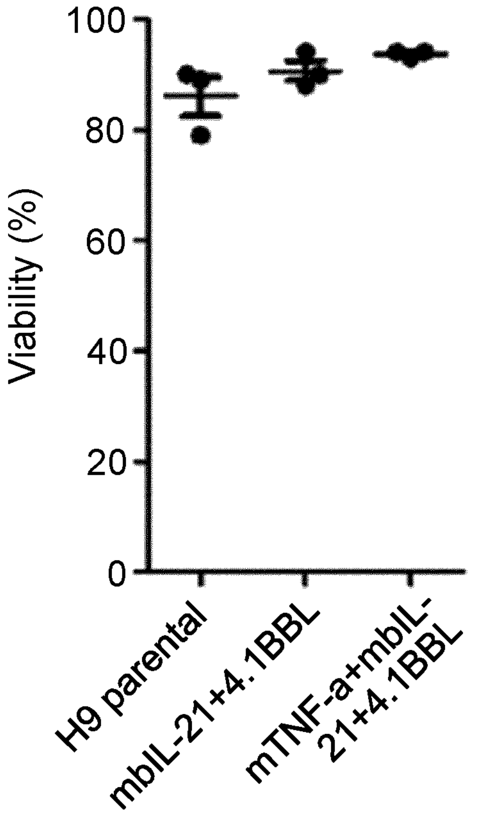
100
80
60
40
20
0
Viability (%)
H9 parental
mbIL-21+4.1BBL
mTNF-a+mbIL-21+4.1BBL

FIG. 3B

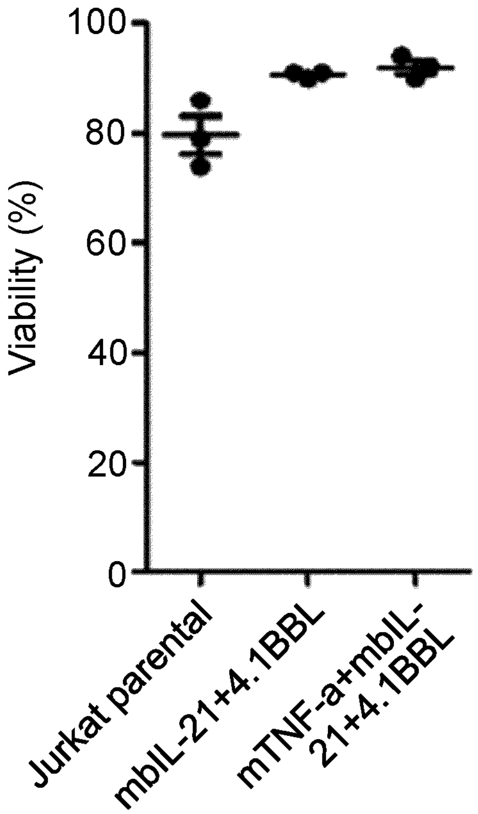
100
80
60
40
20
0
Viability (%)
Jurkat parental
mbIL-21+4.1BBL
mTNF-a+mbIL-21+4.1BBL

FIG. 3C

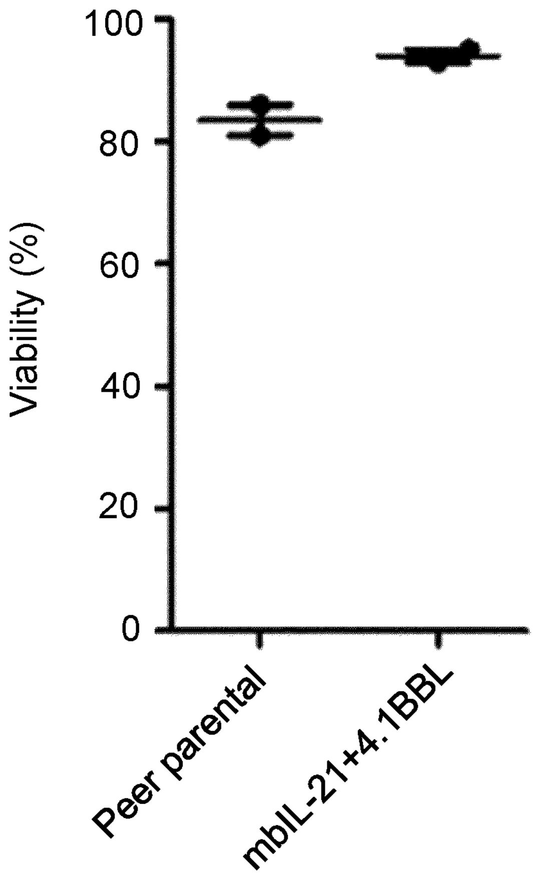
100
80
60
40
20
0
Viability (%)
Peer parental
mbIL-21+4.1BBL

FIG. 3D

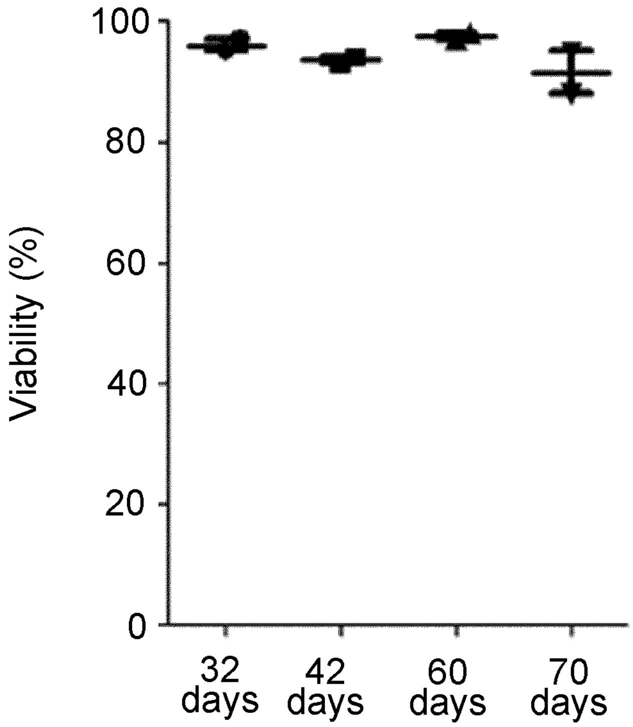
100
80
60
40
20
0
Viability (%)
32 days
42 days
60 days
70 days

FIG. 3E

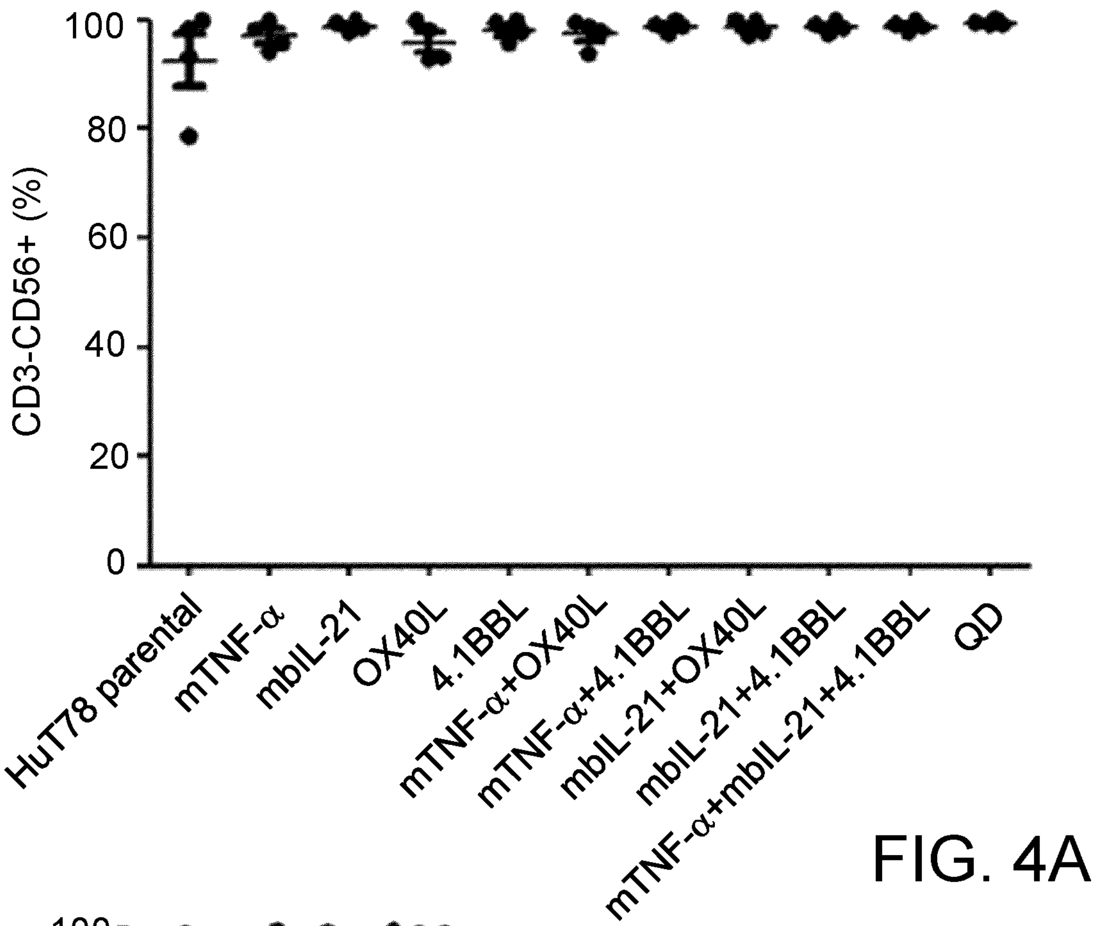
100
80
60
40
20
0
CD3-CD56+ (%)
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 4A

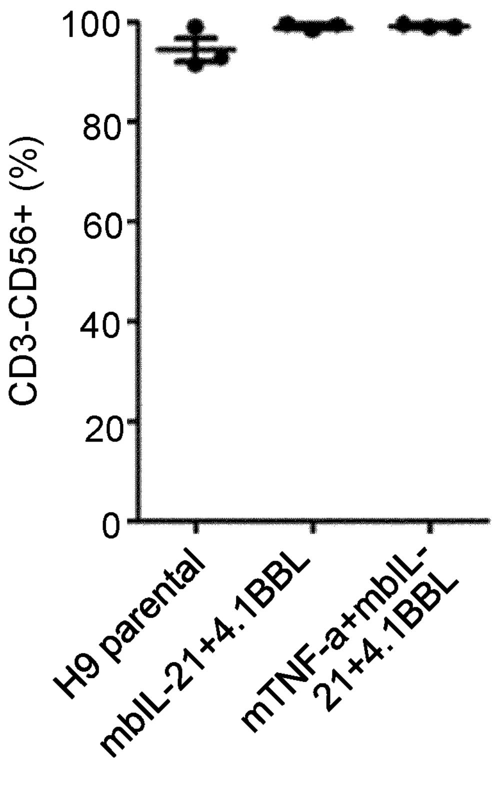
100
80
60
40
20
0
CD3-CD56+ (%)
H9 parental
mbIL-21+4.1BBL
mTNF-a+mbIL-21+4.1BBL

FIG. 4B

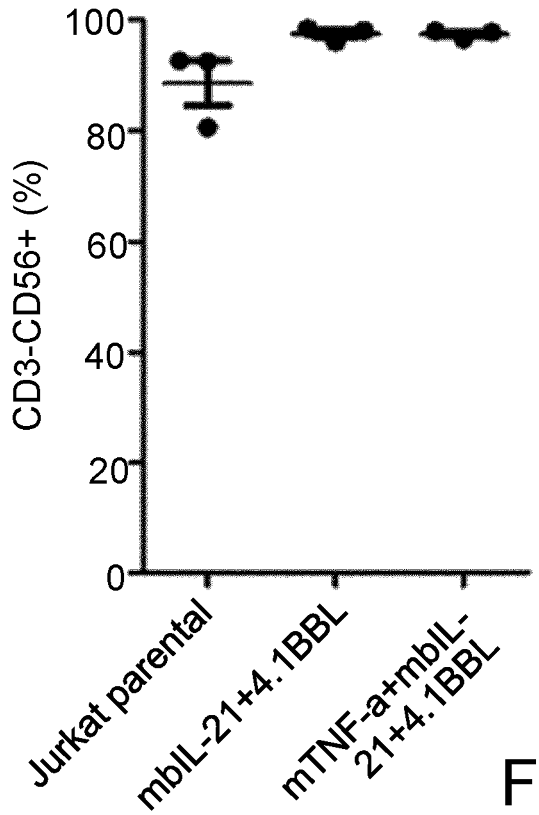
100
80
60
40
20
0
CD3-CD56+ (%)
Jurkat parental
mbIL-21+4.1BBL
mTNF-a+mbIL-21+4.1BBL

FIG. 4C

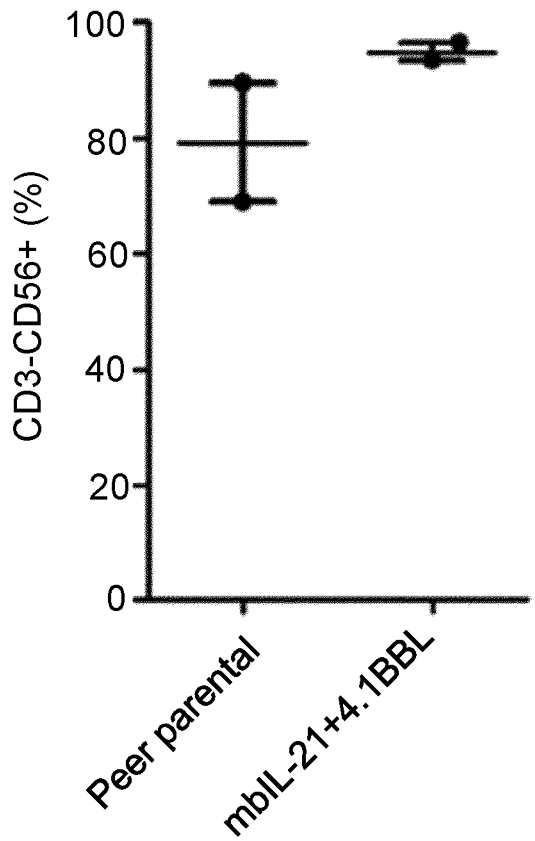
100
80
60
40
20
0
CD3-CD56+ (%)
Peer parental
mbIL-21+4.1BBL

FIG. 4D

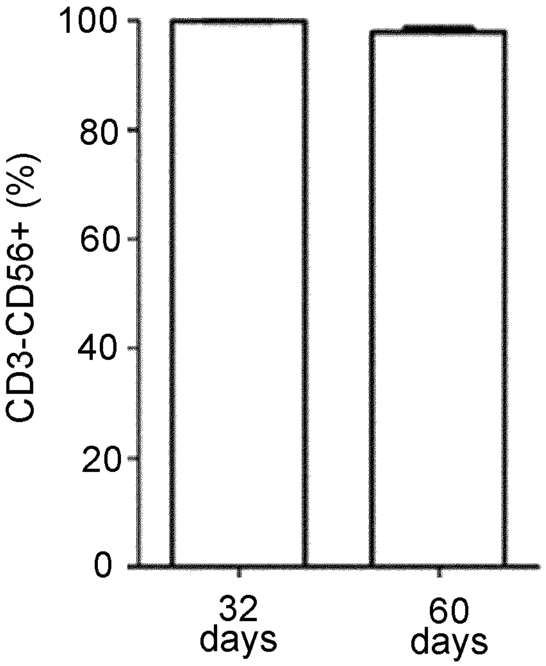

CD3-CD56+ (%)
100
80
60
40
20
0
32 days
60 days


FIG. 4E

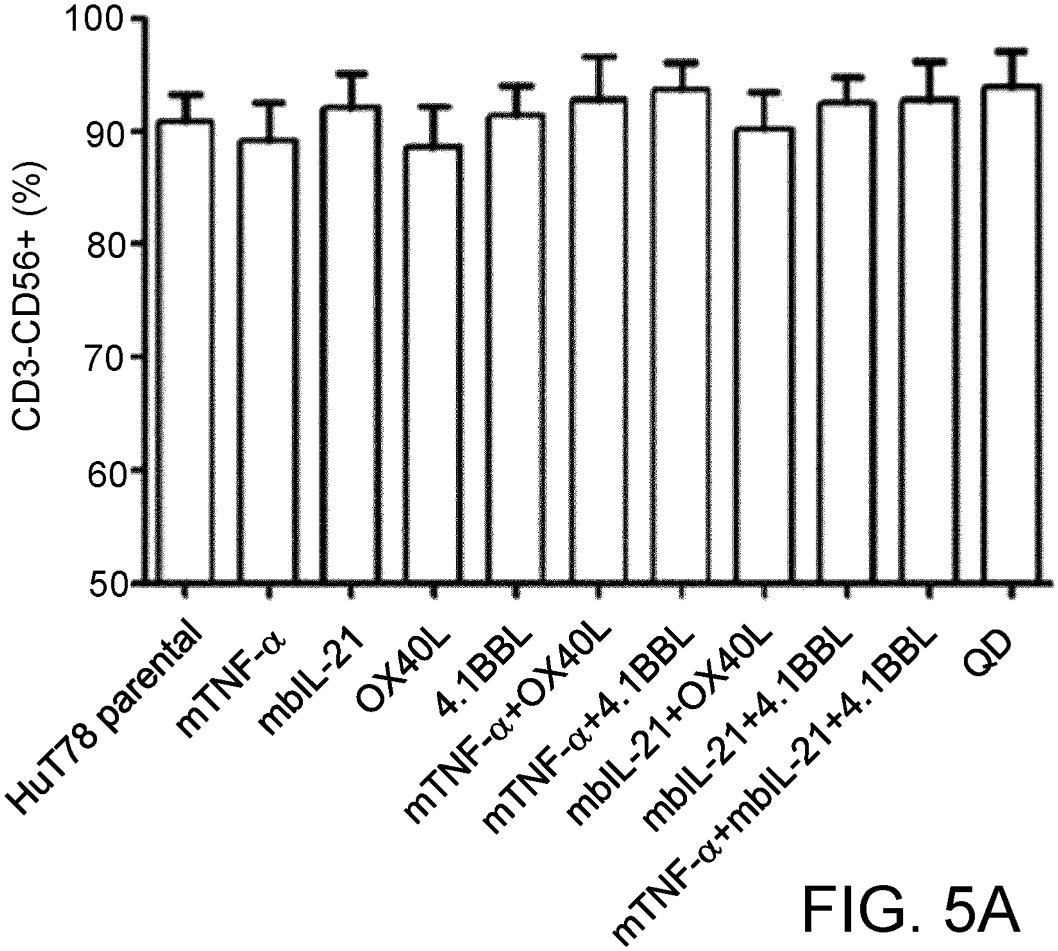

CD3-CD56+ (%)
100
90
80
70
60
50
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD


FIG. 5A

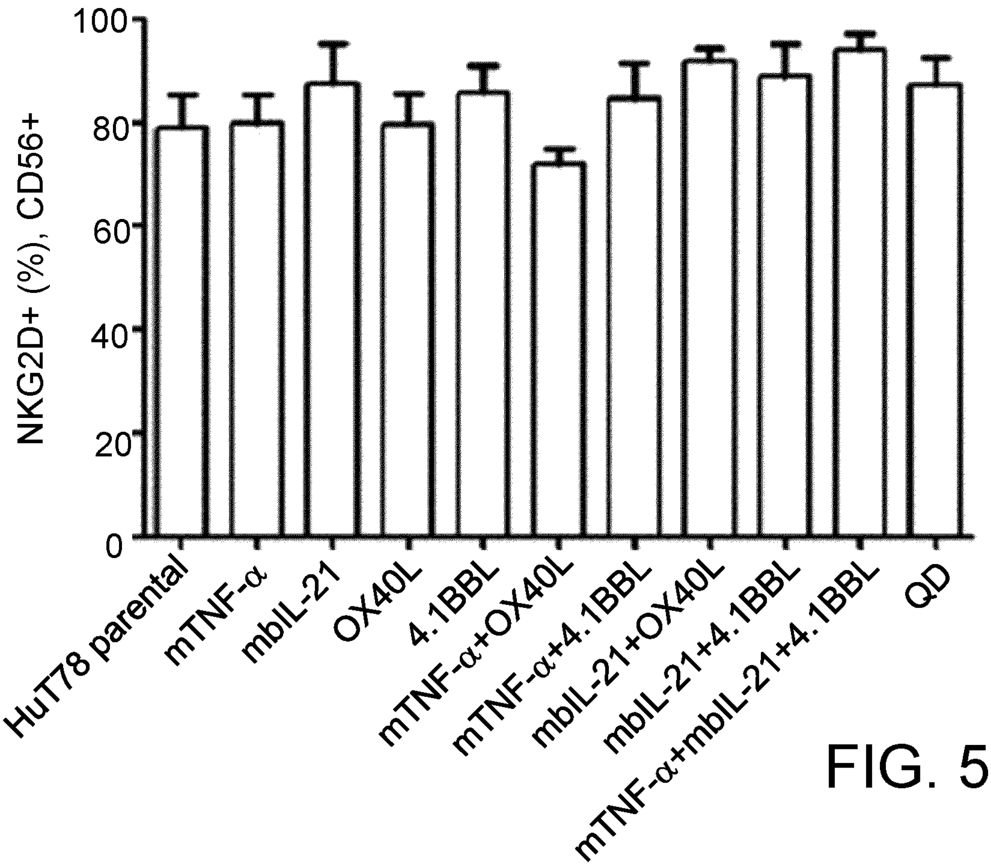
NKG2D+ (%), CD56+
100
80
60
40
20
0
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 5B

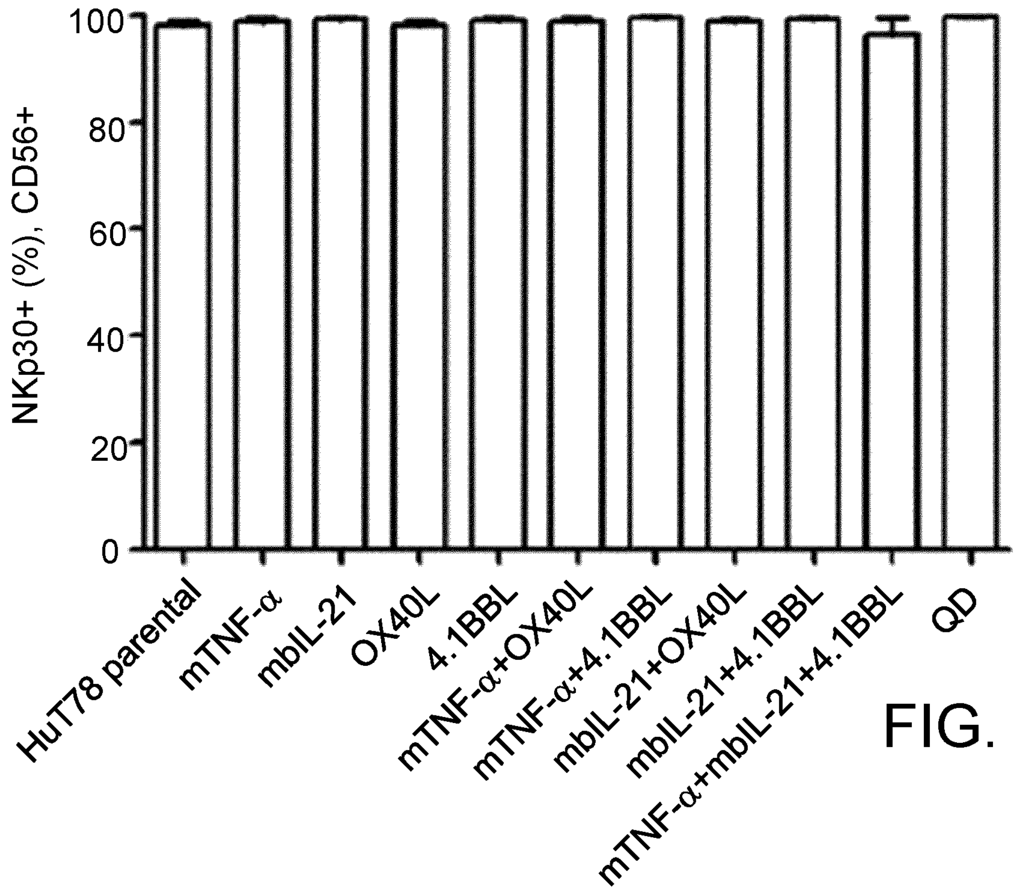
NKp30+ (%), CD56+
100
80
60
40
20
0
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 5C

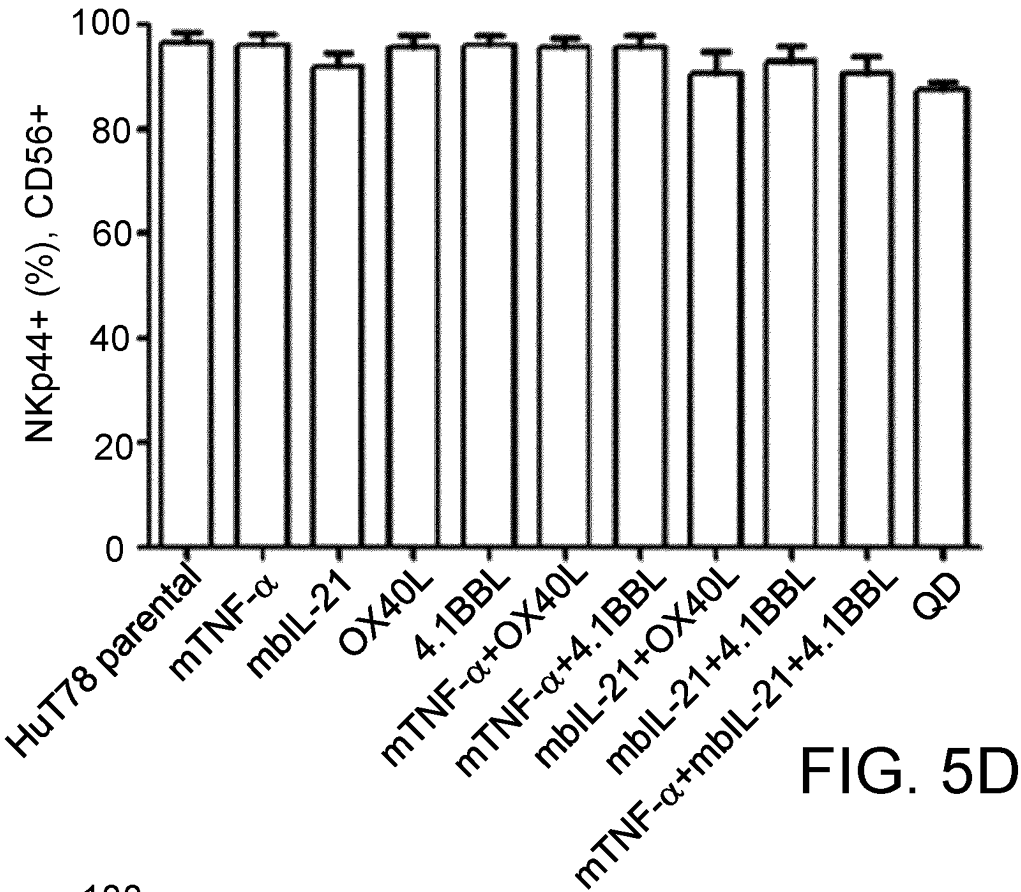
100
80
60
40
20
0
NKp44+ (%), CD56+
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 5D

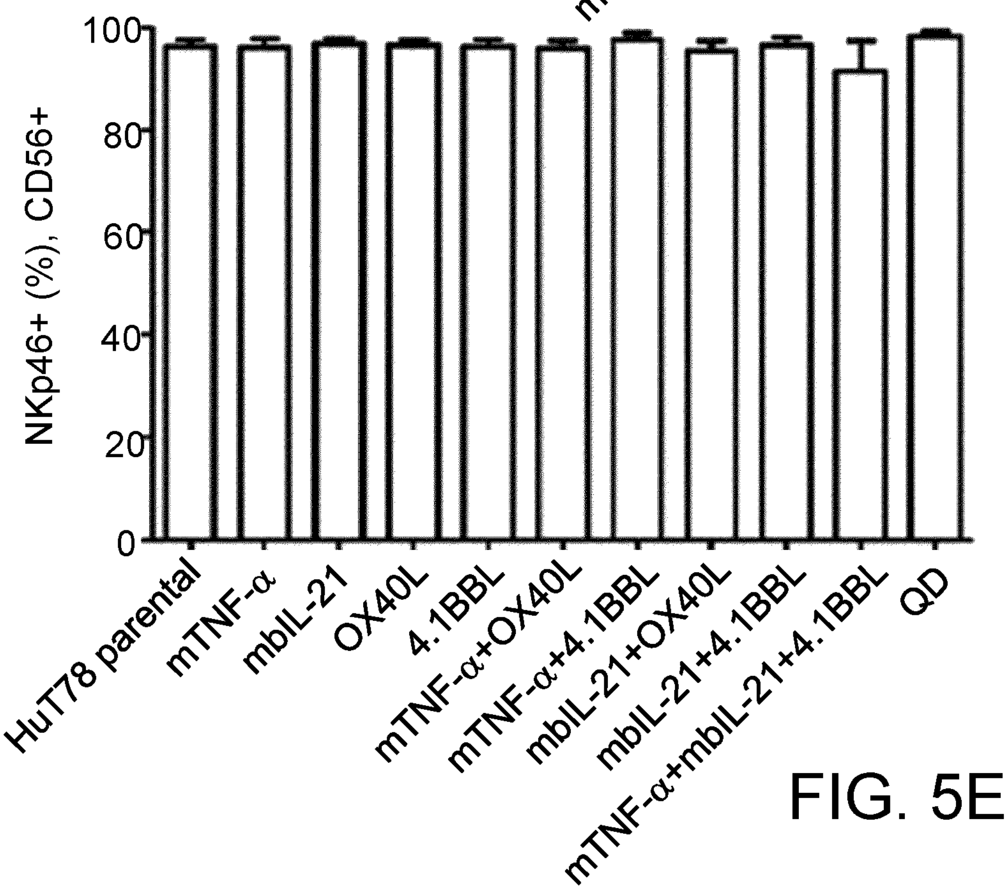
100
80
60
40
20
0
NKp46+ (%), CD56+
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 5E

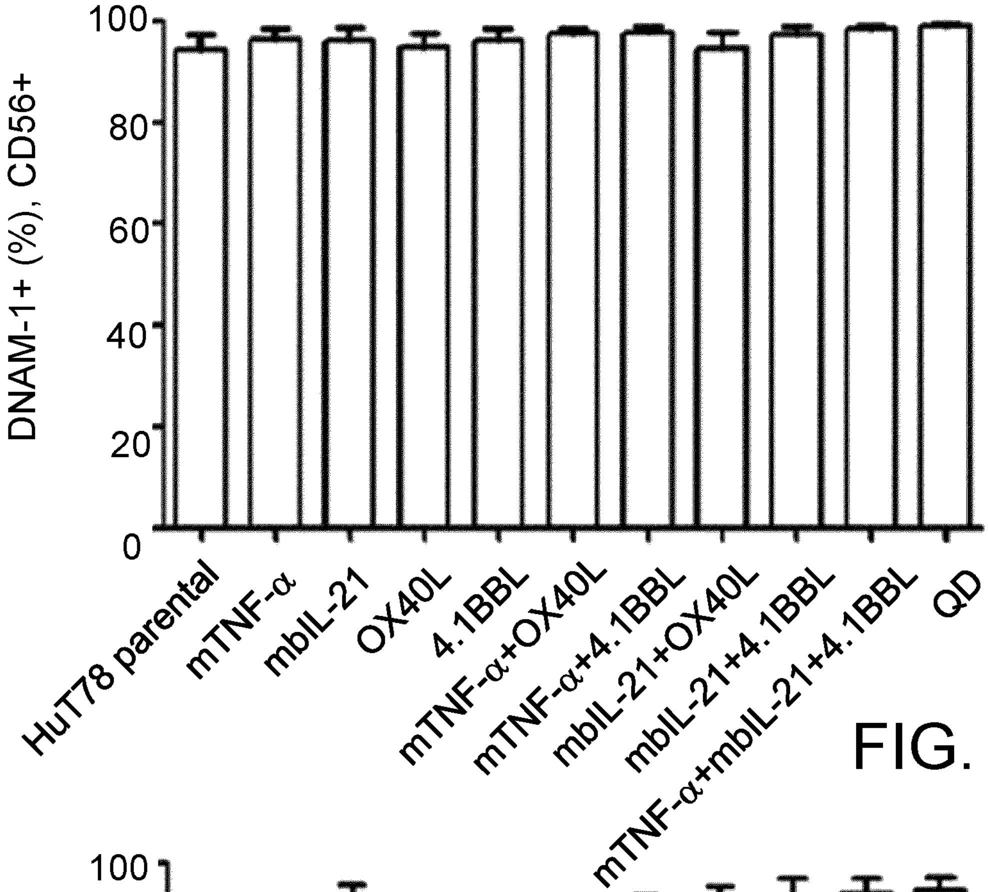
DNAM-1+ (%), CD56+
100
80
60
40
20
0
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 5F

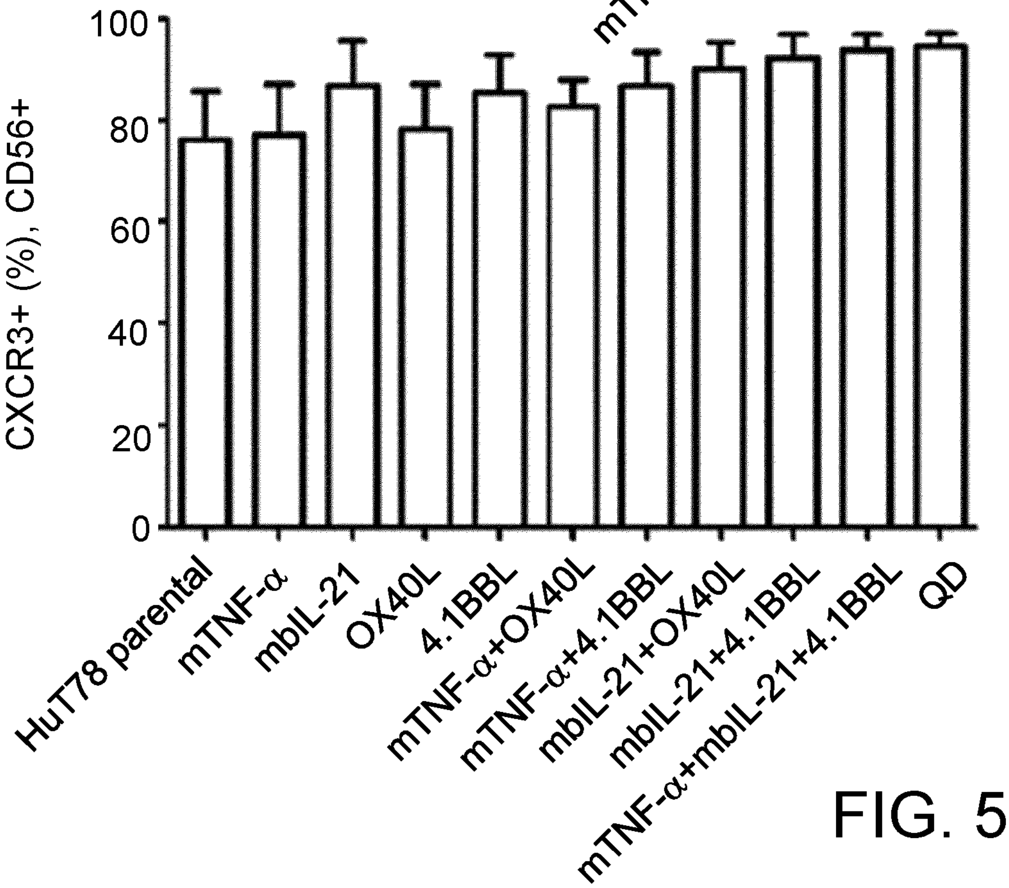
CXCR3+ (%), CD56+
100
80
60
40
20
0
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 5G

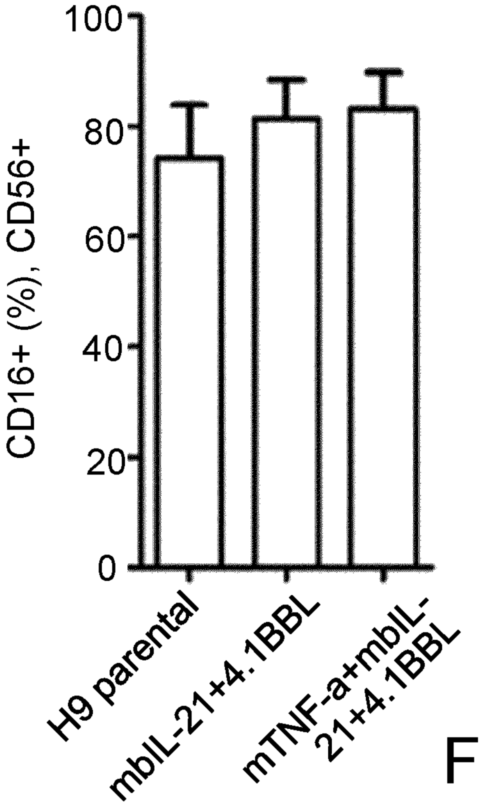
100
80
60
40
20
0
CD16+ (%), CD56+
H9 parental
mbIL-21+4.1BBL
mTNF-a+mbIL-21+4.1BBL

FIG. 6A

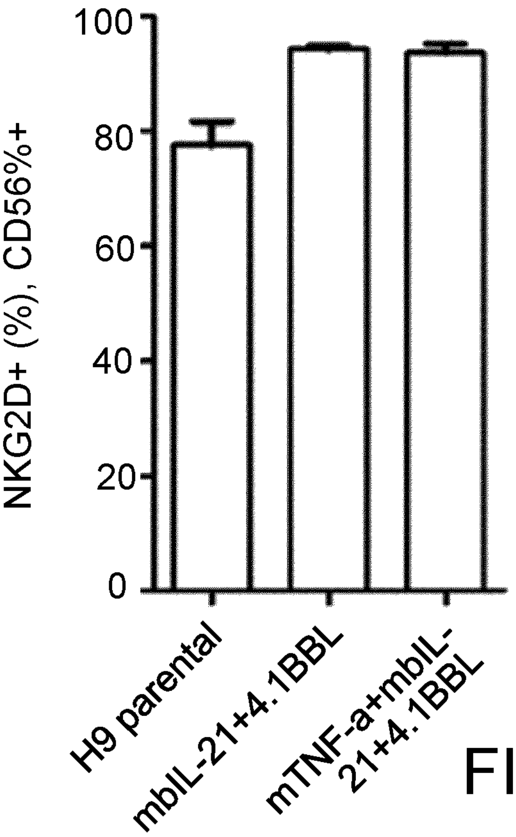
100
80
60
40
20
0
NKG2D+ (%), CD56%+
H9 parental
mbIL-21+4.1BBL
mTNF-a+mbIL-21+4.1BBL

FIG. 6B

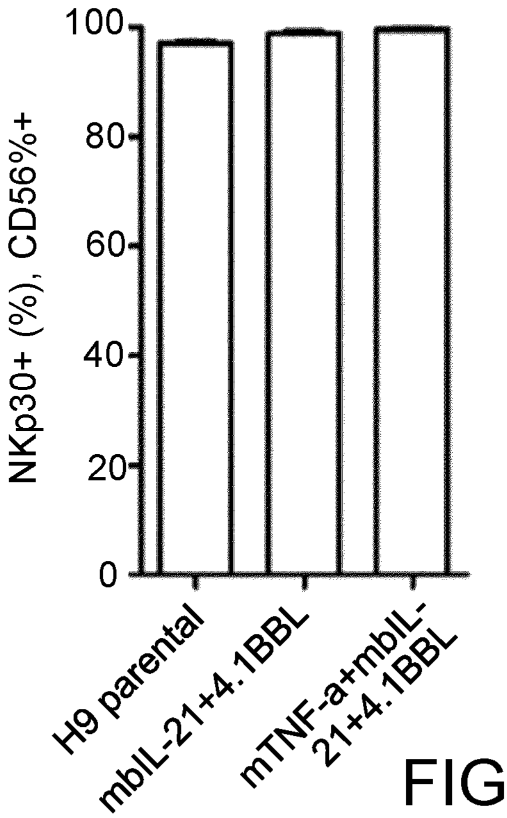
100
80
60
40
20
0
NKp30+ (%), CD56%+
H9 parental
mbIL-21+4.1BBL
mTNF-a+mbIL-21+4.1BBL

FIG. 6C

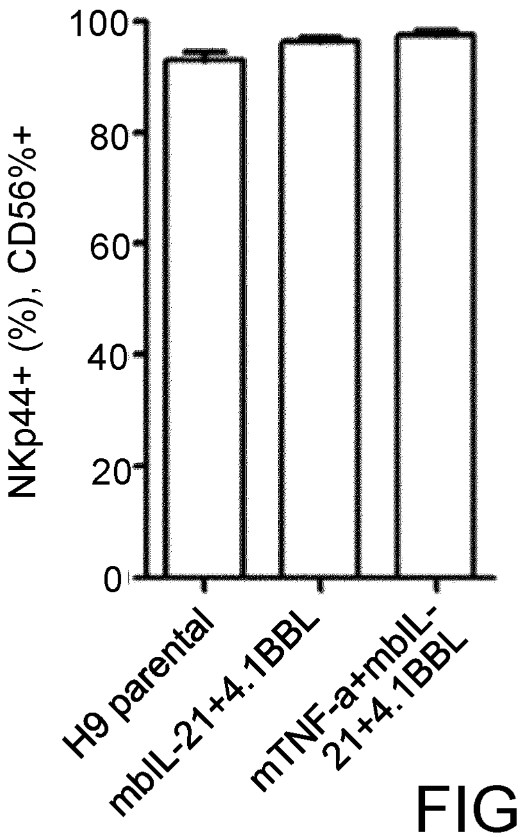
100
80
60
40
20
0
NKp44+ (%), CD56%+
H9 parental
mbIL-21+4.1BBL
mTNF-a+mbIL-21+4.1BBL

FIG. 6D

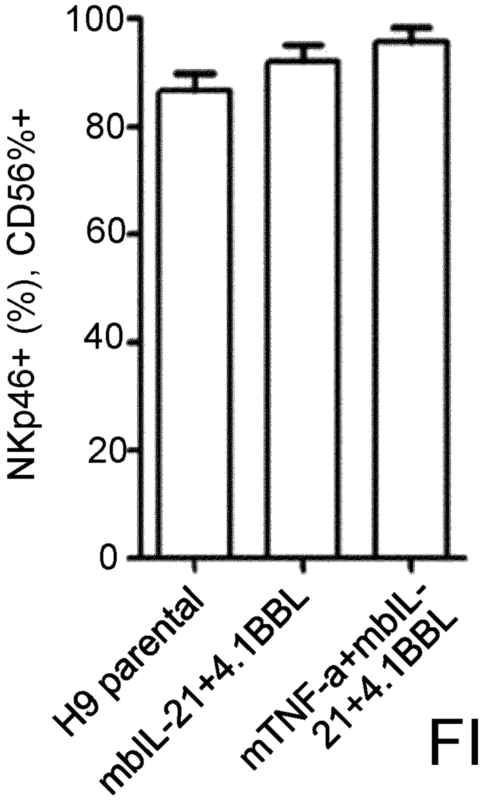
NKp46+ (%), CD56%+
100
80
60
40
20
0
H9 parental
mbIL-21+4.1BBL
mTNF-a+mbIL-21+4.1BBL

FIG. 6E

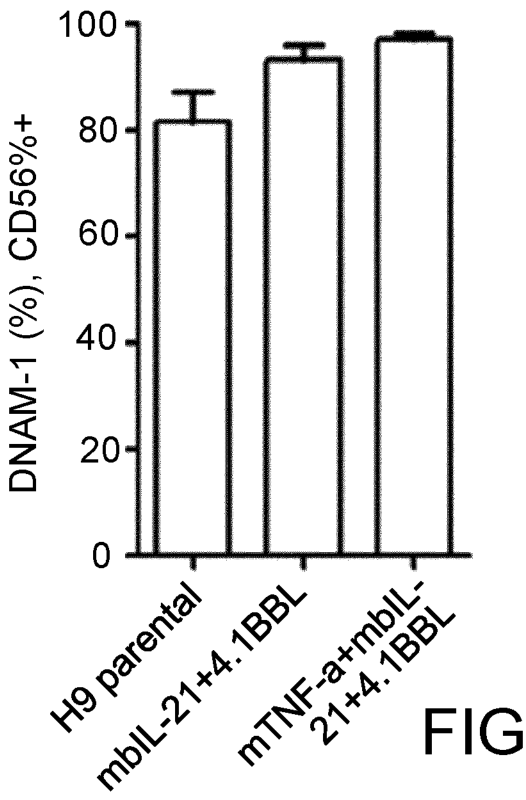
DNAM-1 (%), CD56%+
100
80
60
40
20
0
H9 parental
mbIL-21+4.1BBL
mTNF-a+mbIL-21+4.1BBL

FIG. 6F

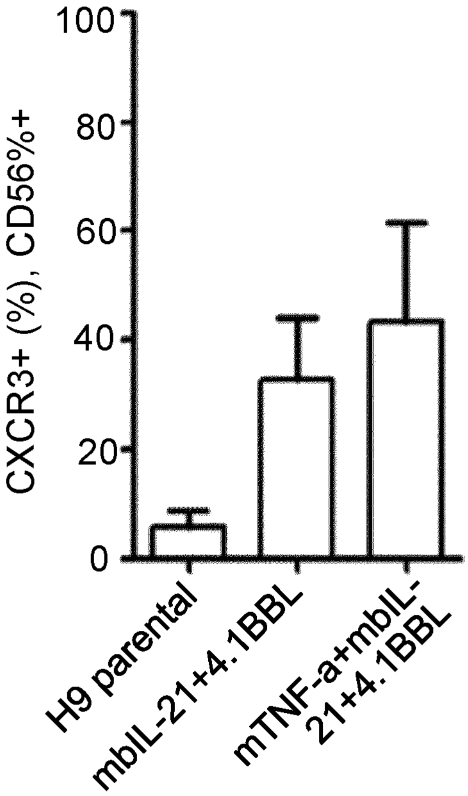
100
80
60
40
20
0
CXCR3+ (%), CD56%+
H9 parental
mbIL-21+4.1BBL
mTNF-a+mbIL-21+4.1BBL

FIG. 6G

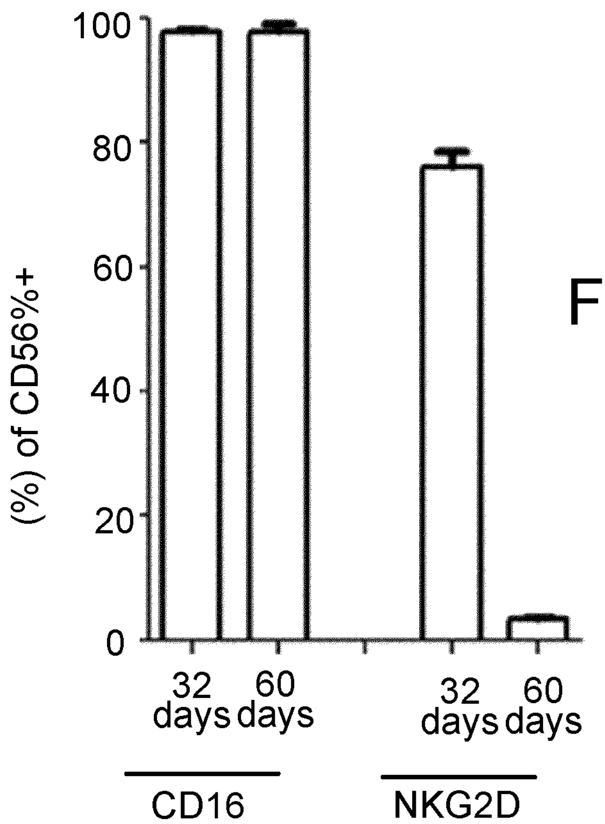
100
80
60
40
20
0
(%) of CD56%+
32 days
60 days
32 days
60 days
CD16
NKG2D

FIG. 7A

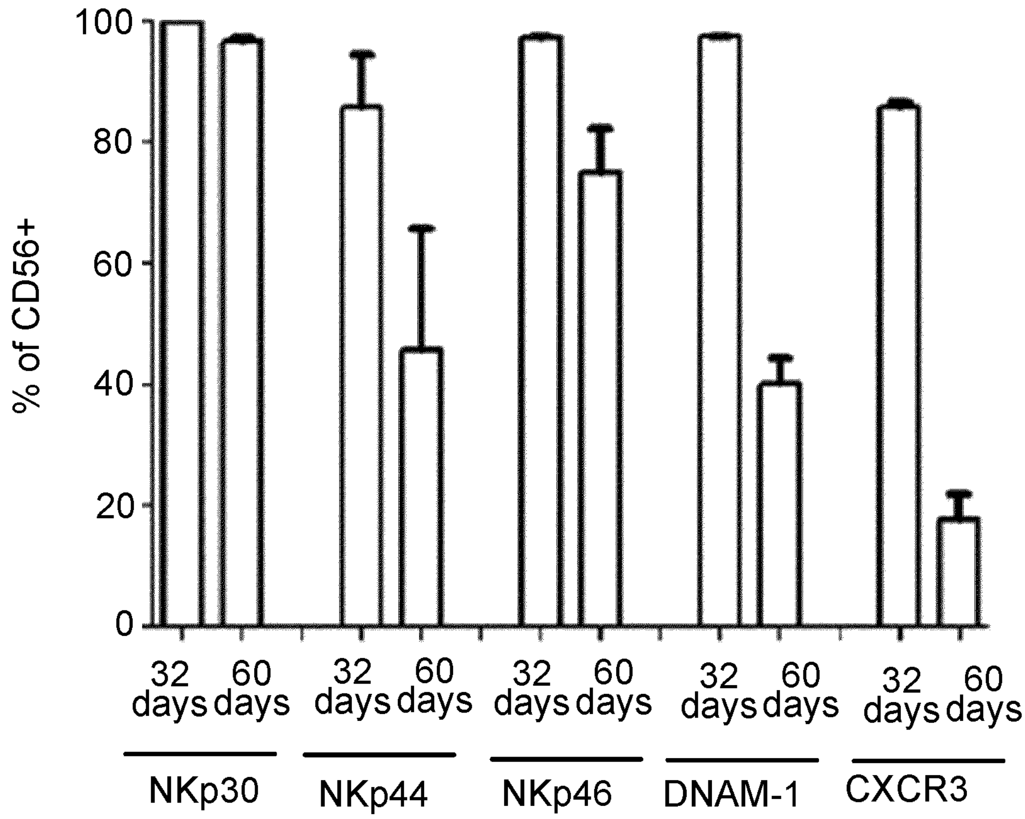
100
80
60
40
20
0
% of CD56+
32 days
60 days
32 days
60 days
32 days
60 days
32 days
60 days
32 days
60 days
NKp30
NKp44
NKp46
DNAM-1
CXCR3

FIG. 7B

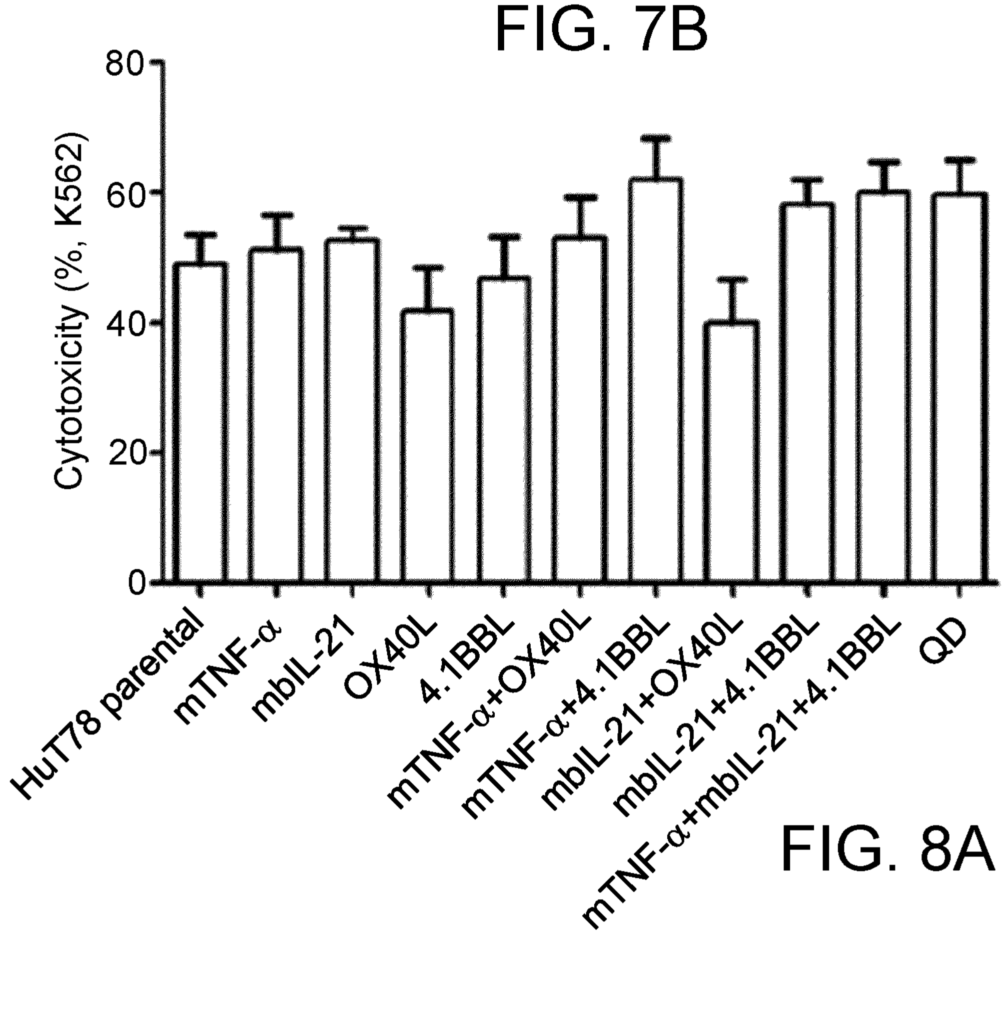
80
60
40
20
0
Cytotoxicity (%, K562)
HuT78 parental
mTNF-α
mbIL-21
OX40L
4.1BBL
mTNF-α+OX40L
mTNF-α+4.1BBL
mbIL-21+OX40L
mbIL-21+4.1BBL
mTNF-α+mbIL-21+4.1BBL
QD

FIG. 8A

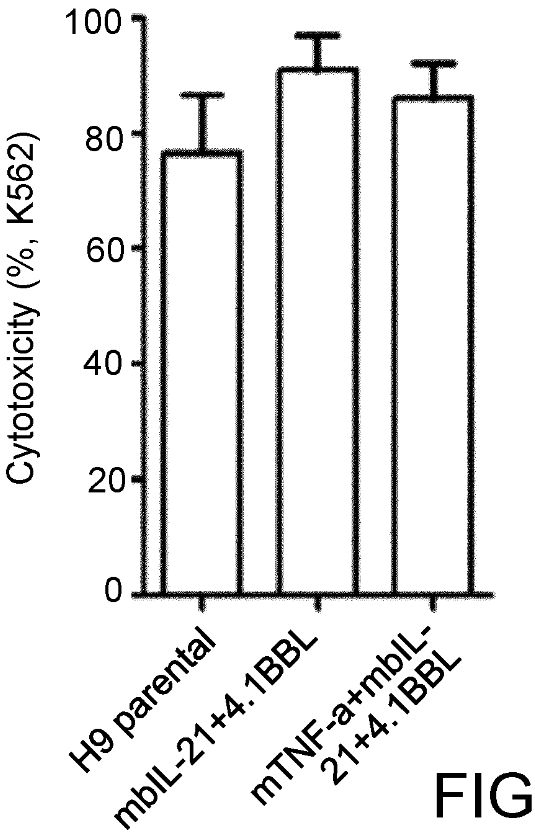
100
80
60
40
20
0
Cytotoxicity (%, K562)
H9 parental
mbIL-21+4.1BBL
mTNF-a+mbIL-21+4.1BBL

FIG. 8B

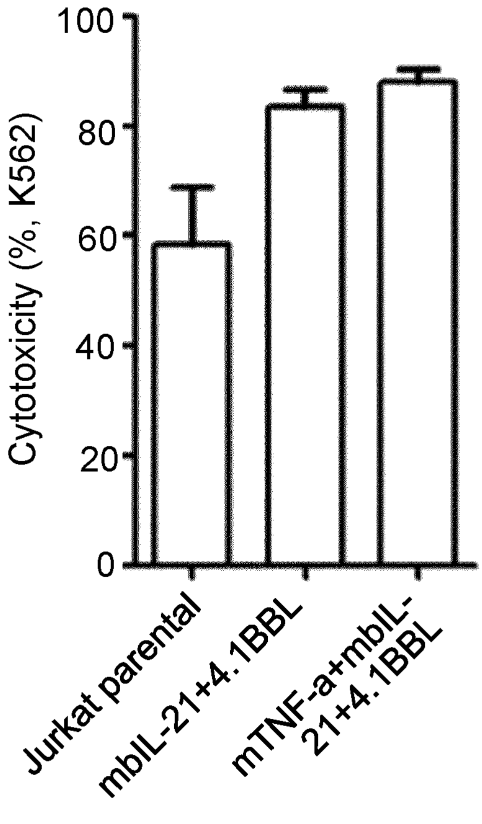
100
80
60
40
20
0
Cytotoxicity (%, K562)
Jurkat parental
mbIL-21+4.1BBL
mTNF-a+mbIL-21+4.1BBL

FIG. 8C

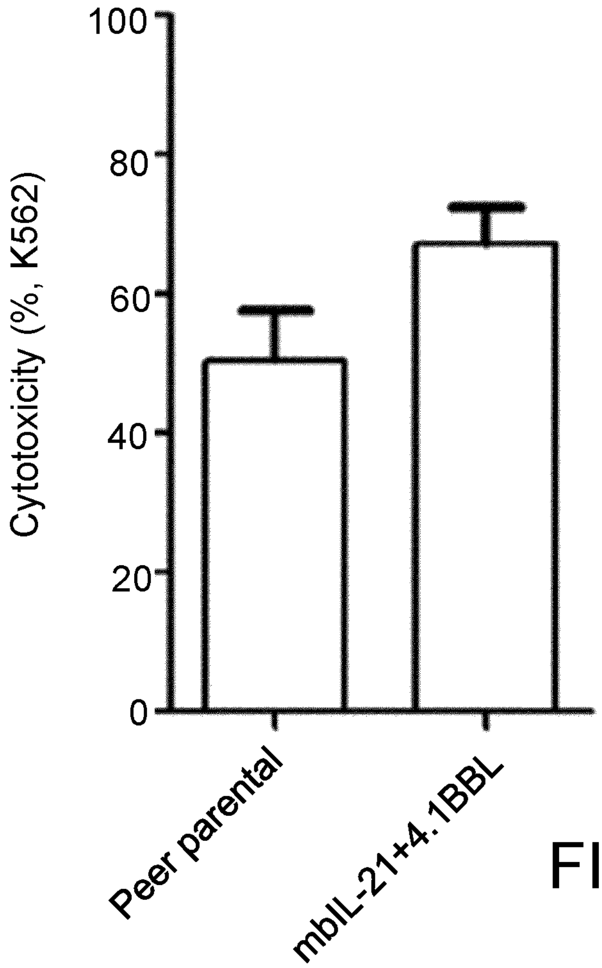
100
80
60
40
20
0
Cytotoxicity (%, K562)
Peer parental
mbIL-21+4.1BBL

FIG. 8D

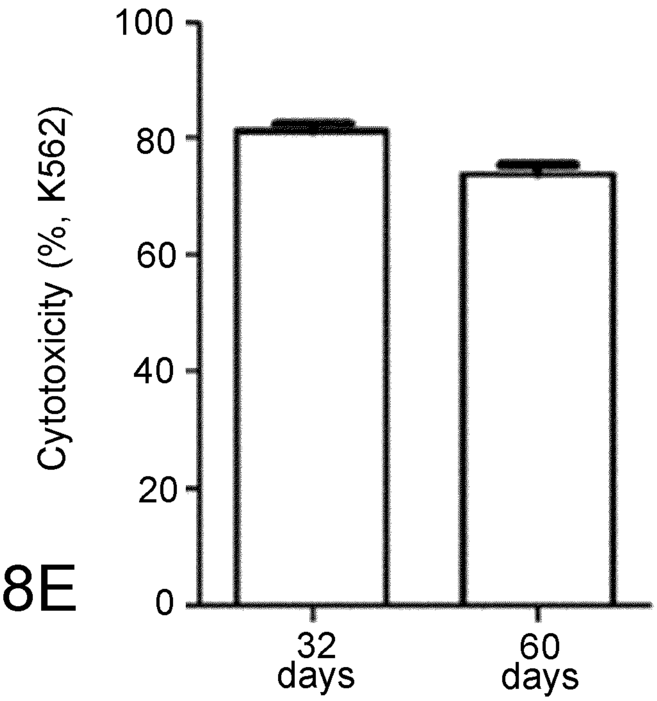
100
80
60
40
20
0
Cytotoxicity (%, K562)
32 days
60 days

FIG. 8E

Animal model: Raji (cancer cell line)

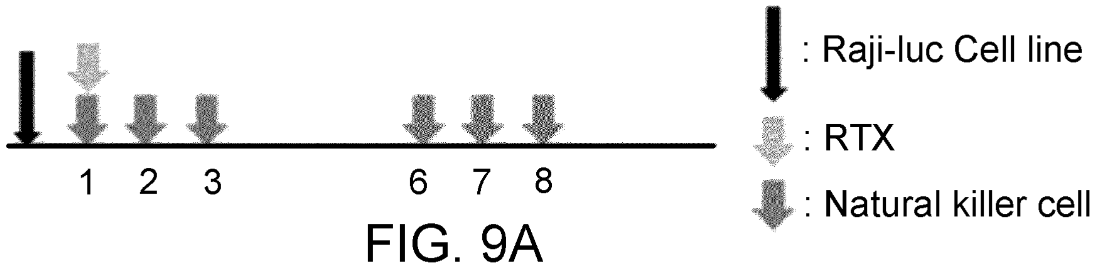


FIG. 9A

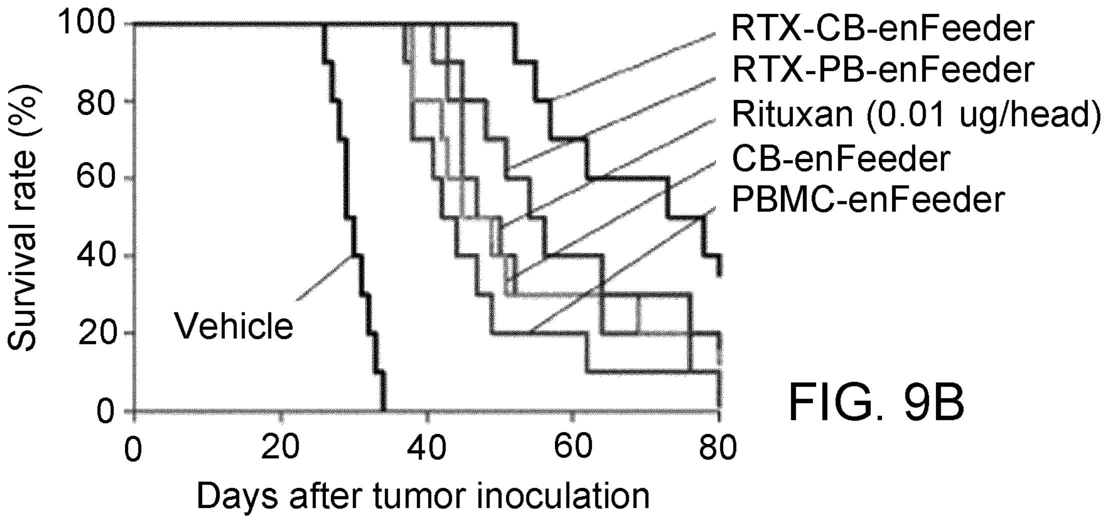


FIG. 9B

Animal model: Ramos (cancer cell line)

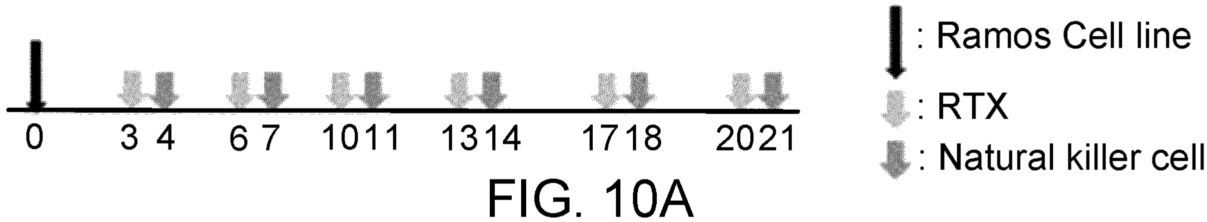


FIG. 10A

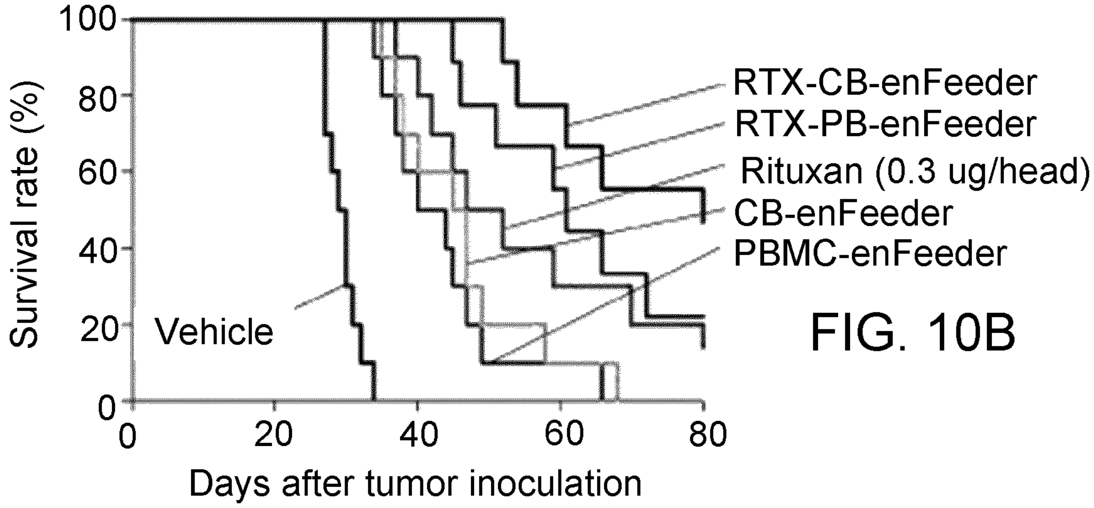


FIG. 10B

**INTERNATIONAL SEARCH REPORT**

International application No.
**PCT/KR2019/015469**

A. CLASSIFICATION OF SUBJECT MATTER

***C12N 5/0783(2010.01)i***

According to International Patent Classification (IPC) or to both national classification and IPC

B. FIELDS SEARCHED

Minimum documentation searched (classification system followed by classification symbols)
C12N 5/0783; A01N 63/00; A61K 35/12; A61K 35/14; C12N 5/10

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: NK cell, feeder cell, seed cell, culturing, CD4+ T cell, 4-1BBL, mbIL-21, OX40L, mTNF-alpha

C. DOCUMENTS CONSIDERED TO BE RELEVANT

| Category* | Citation of document, with indication, where appropriate, of the relevant passages | Relevant to claim No. |
|---|---|---|
| X | MIN, Bokyung. Identification of NK cell costimulatory receptors for large-scale expansion of NK cells for adoptive immunotherapy in cancer patients. 한국과학기술원 박사학위논문 (Ph.D. thesis. Korea Advanced Institute of Science and Technology). May 2018, pages 1-143<br>See abstract; pages 29-100, 116-119. | 1-26 |
| Y | KR 10-2016-0063114 A (GREEN CROSS LAB CELL CORPORATION et al.) 03 June 2016<br>See abstract; claims 1-22. | 1-8,11-13,16-26 |
| Y | CN 102911918 A (SHANGHAI JIAOTONG UNIVERSITY SCHOOL OF MEDICINE) 06 February 2013<br>See abstract; claims 1-9, 16-1. | 1-8,11-13,16-26 |
| Y | EP 2856876 A1 (MEMORIAL SLOAN-KETTERING CANCER CENTER) 08 April 2015<br>See abstract; claims 1-4, 8-18; paragraphs [0012], [0070]-[0071], [0077]-[0077]. | 7-8 |
| A | KR 10-1520534 B1 (KOREA UNIVERSITY RESEARCH AND BUSINESS FOUNDATION) 21 May 2015<br>See the entire document. | 1-26 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 FEBRUARY 2020 (24.02.2020) | **25 FEBRUARY 2020 (25.02.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2019/015469**

| Patent document cited in search report | Publication date | Patent family member | Publication date |
|---|---|---|---|
| KR 10-2016-0063114 A | 03/06/2016 | AU 2015-354941 A1 | 01/06/2017 |
| | | AU 2015-354941 B2 | 15/11/2018 |
| | | CN 107002039 A | 01/08/2017 |
| | | EP 3224349 A1 | 04/10/2017 |
| | | JP 2018-501779 A | 25/01/2018 |
| | | KR 10-1799986 B1 | 21/11/2017 |
| | | US 2017-0319621 A1 | 09/11/2017 |
| | | WO 2016-085248 A1 | 02/06/2016 |
| CN 102911918 A | 06/02/2013 | CN 102559600 A | 11/07/2012 |
| | | CN 102911918 B | 10/12/2014 |
| EP 2856876 A1 | 08/04/2015 | AU 2008-233051 A1 | 09/10/2008 |
| | | AU 2014-203686 A1 | 24/07/2014 |
| | | CA 2682527 A1 | 09/10/2008 |
| | | CA 2967847 A1 | 09/10/2008 |
| | | DK 2141997 T3 | 11/02/2013 |
| | | DK 2537416 T3 | 05/01/2015 |
| | | DK 2856876 T3 | 03/04/2018 |
| | | EP 2141997 A1 | 13/01/2010 |
| | | EP 2141997 B1 | 31/10/2012 |
| | | EP 2537416 A1 | 26/12/2012 |
| | | EP 2537416 B1 | 12/11/2014 |
| | | EP 2856876 B1 | 31/01/2018 |
| | | EP 3357338 A1 | 08/08/2018 |
| | | ES 2398760 T3 | 21/03/2013 |
| | | HK 1180183 A1 | 02/10/2015 |
| | | HK 1208996 A1 | 24/03/2016 |
| | | JP 2010-523083 A | 15/07/2010 |
| | | JP 2016-019521 A | 04/02/2016 |
| | | JP 2018-050620 A | 05/04/2018 |
| | | JP 2020-005648 A | 16/01/2020 |
| | | JP 5840837 B2 | 06/01/2016 |
| | | JP 6215265 B2 | 18/10/2017 |
| | | JP 6584466 B2 | 02/10/2019 |
| | | NO 2856876 T3 | 30/06/2018 |
| | | PL 2856876 T3 | 29/06/2018 |
| | | US 10117897 B2 | 06/11/2018 |
| | | US 2010-0178276 A1 | 15/07/2010 |
| | | US 2013-0121960 A1 | 16/05/2013 |
| | | US 2016-0008398 A1 | 14/01/2016 |
| | | US 2019-0000881 A1 | 03/01/2019 |
| | | US 8252592 B2 | 28/08/2012 |
| | | US 8389282 B2 | 05/03/2013 |
| | | US 9220728 B2 | 29/12/2015 |
| | | WO 2008-121420 A1 | 09/10/2008 |
| KR 10-1520534 B1 | 21/05/2015 | CN 104321425 A | 28/01/2015 |
| | | CN 104321425 B | 10/08/2018 |

Form PCT/ISA/210 (patent family annex) (January 2015)

**INTERNATIONAL SEARCH REPORT**
Information on patent family members

International application No.
**PCT/KR2019/015469**

| Patent document cited in search report | Publication date | Patent family member | Publication date |
|---|---|---|---|
| | | KR 10-1643165 B1 | 05/08/2016 |
| | | KR 10-2015-0039592 A | 10/04/2015 |
| | | US 2015-0152387 A1 | 04/06/2015 |
| | | US 2018-0223257 A1 | 09/08/2018 |
| | | US 9938498 B2 | 10/04/2018 |
| | | WO 2013-168978 A1 | 14/11/2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BIOSSEL L. et al.** *Biology of Blood and Marrow Transplantation,* 2008, vol. 14, 1031-1038
- **FIAS A.M. et al.** *Experimental Hematology,* 2008, vol. 36 (1), 61-68
- **GOODIER et al.** *J. Immunol.,* 2000, vol. 165 (1), 139-147
- **CONDIOTTI et al.** *Experimental Hematol.,* 2001, vol. 29 (1), 104-113
- **FUJISAKI et al.** *Cancer Res.,* 2009, vol. 69 (9), 4010-4017
- **GONG et al.** *Tissue Antigens,* 2010, vol. 76 (6), 467-475

<110> Green Cross Lab Cell Corporation

<120> METHOD FOR CULTURING NATURAL KILLER CELLS DERIVED FROM CORD BLOODS WITH GENETICALLY ENGINEERED T CELLS

<130> FPD/201810-0021

<160> 25

<170> KoPatentIn 3.0

<210> 1
<211> 254
<212> PRT
<213> Artificial Sequence

<220>
<223> amino acid seqeunce for 4-1BBL

<400> 1
Met Glu Tyr Ala Ser Asp Ala Ser Leu Asp Pro Glu Ala Pro Trp Pro
1 5 10 15

Pro Ala Pro Arg Ala Arg Ala Cys Arg Val Leu Pro Trp Ala Leu Val
20 25 30

Ala Gly Leu Leu Leu Leu Leu Leu Leu Ala Ala Ala Cys Ala Val Phe
35 40 45

Leu Ala Cys Pro Trp Ala Val Ser Gly Ala Arg Ala Ser Pro Gly Ser
50 55 60

Ala Ala Ser Pro Arg Leu Arg Glu Gly Pro Glu Leu Ser Pro Asp Asp
65 70 75 80

Pro Ala Gly Leu Leu Asp Leu Arg Gln Gly Met Phe Ala Gln Leu Val
85 90 95

Ala Gln Asn Val Leu Leu Ile Asp Gly Pro Leu Ser Trp Tyr Ser Asp
100 105 110

Pro Gly Leu Ala Gly Val Ser Leu Thr Gly Gly Leu Ser Tyr Lys Glu
115 120 125

Asp Thr Lys Glu Leu Val Val Ala Lys Ala Gly Val Tyr Tyr Val Phe
130 135 140

Phe Gln Leu Glu Leu Arg Arg Val Val Ala Gly Glu Gly Ser Gly Ser
145 150 155 160

Val Ser Leu Ala Leu His Leu Gln Pro Leu Arg Ser Ala Ala Gly Ala
165 170 175

Ala Ala Leu Ala Leu Thr Val Asp Leu Pro Pro Ala Ser Ser Glu Ala
180 185 190

Arg Asn Ser Ala Phe Gly Phe Gln Gly Arg Leu Leu His Leu Ser Ala
195 200 205

Gly Gln Arg Leu Gly Val His Leu His Thr Glu Ala Arg Ala Arg His
210 215 220

```
Ala Trp Gln Leu Thr Gln Gly Ala Thr Val Leu Gly Leu Phe Arg Val
225                 230                 235                 240

Thr Pro Glu Ile Pro Ala Gly Leu Pro Ser Pro Arg Ser Glu
                245                 250


<210>   2
<211>   765
<212>   DNA
<213>   Artificial Sequence

<220>
<223>   nucleotide sequence of 4-1BBL


<400>   2
atggaatacg cctctgacgc ttcactggac cccgaagccc cgtggcctcc cgcgccccgc        60

gctcgcgcct gccgcgtact gccttgggcc ctggtcgcgg ggctgctgct gctgctgctg       120

ctcgctgccg cctgcgccgt cttcctcgcc tgcccctggg ccgtgtccgg ggctcgcgcc       180

tcgcccggct ccgcggccag cccgagactc cgcgagggtc ccgagctttc gcccgacgat       240

cccgccggcc tcttggacct gcggcagggc atgtttgcgc agctggtggc ccaaaatgtt       300

ctgctgatcg atgggcccct gagctggtac agtgacccag gcctggcagg cgtgtccctg       360

acgggggggcc tgagctacaa agaggacacg aaggagctgg tggtggccaa ggctggagtc      420

tactatgtct tctttcaact agagctgcgg cgcgtggtgg ccggcgaggg ctcaggctcc       480

gtttcacttg cgctgcacct gcagccactg cgctctgctg ctggggccgc cgccctggct       540

ttgaccgtgg acctgccacc cgcctcctcc gaggctcgga actcggcctt cggtttccag       600

ggccgcttgc tgcacctgag tgccggccag cgcctgggcg tccatcttca cactgaggcc       660

agggcacgcc atgcctggca gcttacccag ggcgccacag tcttgggact cttccgggtg       720

acccccgaaa tcccagccgg actcccttca ccgaggtcgg aataa                        765


<210>   3
<211>   223
<212>   PRT
<213>   Artificial Sequence

<220>
<223>   amino acid seqeunce for membrane bound IL-21 (mbIL-21)


<400>   3
Met Ala Leu Pro Val Thr Ala Leu Leu Leu Pro Leu Ala Leu Leu Leu
  1               5                  10                  15

His Ala Ala Arg Pro Gln Asp Arg His Met Ile Arg Met Arg Gln Leu
             20                  25                  30

Ile Asp Ile Val Asp Gln Leu Lys Asn Tyr Val Asn Asp Leu Val Pro
        35                  40                  45
```

```
Glu Phe Leu Pro Ala Pro Glu Asp Val Glu Thr Asn Cys Glu Trp Ser
    50                  55                  60

Ala Phe Ser Cys Phe Gln Lys Ala Gln Leu Lys Ser Ala Asn Thr Gly
65                  70                  75                  80

Asn Asn Glu Arg Ile Ile Asn Val Ser Ile Lys Lys Leu Lys Arg Lys
                85                  90                  95

Pro Pro Ser Thr Asn Ala Gly Arg Arg Gln Lys His Arg Leu Thr Cys
            100                 105                 110

Pro Ser Cys Asp Ser Tyr Glu Lys Lys Pro Pro Lys Glu Phe Leu Glu
        115                 120                 125

Arg Phe Lys Ser Leu Leu Gln Lys Met Ile His Gln His Leu Ser Ser
    130                 135                 140

Arg Thr His Gly Ser Glu Asp Ser Ala Lys Pro Thr Thr Thr Pro Ala
145                 150                 155                 160

Pro Arg Pro Pro Thr Pro Ala Pro Thr Ile Ala Ser Gln Pro Leu Ser
                165                 170                 175

Leu Arg Pro Glu Ala Cys Arg Pro Ala Ala Gly Gly Ala Val His Thr
            180                 185                 190

Arg Gly Leu Asp Phe Ala Cys Asp Ile Tyr Ile Trp Ala Pro Leu Ala
        195                 200                 205

Gly Thr Cys Gly Val Leu Leu Leu Ser Leu Val Ile Thr Leu Tyr
    210                 215                 220
```

<210> 4
<211> 672
<212> DNA
<213> Artificial Sequence

<220>
<223> nucleotide sequence of membrane bound IL-21 (mbIL-21)

<400> 4

```
atggctctgc ccgtcaccgc tctgctgctg cctctggccc tgctgctgca cgccgcaaga        60

ccacaggacc gccacatgat tcggatgagg cagctgatcg acattgtgga tcagctgaag       120

aactacgtga atgacctggt ccccgagttc ctgcccgctc ctgaggatgt cgaaacaaac       180

tgcgaatggt ctgcattcag ttgttttcag aaggctcagc tgaaatctgc aaacactgga       240

aacaatgagc gaatcattaa tgtgagcatc aagaaactga agcggaaacc cccttccact       300

aatgcaggcc ggagacagaa gcaccgactg acctgcccct catgtgacag ctatgaaaag       360

aaaccaccca aagagttcct ggagcggttc aagagcctgc tccagaaaat gatccaccag       420

cacctgagca gccggaccca cggcagcgag gattctgcca agcctaccac aactccagct       480

ccccgccctc caacccctgc accaacaatt gccagtcagc ccctgtcact gaggcctgaa       540
```

```
gcatgccgcc cagccgctgg cggagcagtc cacacacgag gcctggactt tgcttgtgat        600

atctacattt gggcacctct ggctggaact tgcggcgtcc tgctgctgtc cctggtcatc        660

accctgtatt ga                                                            672


<210>    5
<211>    183
<212>    PRT
<213>    Artificial Sequence

<220>
<223>    amino acid seqeunce for OX40L


<400>    5
Met Glu Arg Val Gln Pro Leu Glu Glu Asn Val Gly Asn Ala Ala Arg
1               5                   10                  15

Pro Arg Phe Glu Arg Asn Lys Leu Leu Leu Val Ala Ser Val Ile Gln
            20                  25                  30

Gly Leu Gly Leu Leu Leu Cys Phe Thr Tyr Ile Cys Leu His Phe Ser
        35                  40                  45

Ala Leu Gln Val Ser His Arg Tyr Pro Arg Ile Gln Ser Ile Lys Val
    50                  55                  60

Gln Phe Thr Glu Tyr Lys Lys Glu Lys Gly Phe Ile Leu Thr Ser Gln
65                  70                  75                  80

Lys Glu Asp Glu Ile Met Lys Val Gln Asn Asn Ser Val Ile Ile Asn
                85                  90                  95

Cys Asp Gly Phe Tyr Leu Ile Ser Leu Lys Gly Tyr Phe Ser Gln Glu
            100                 105                 110

Val Asn Ile Ser Leu His Tyr Gln Lys Asp Glu Glu Pro Leu Phe Gln
        115                 120                 125

Leu Lys Lys Val Arg Ser Val Asn Ser Leu Met Val Ala Ser Leu Thr
    130                 135                 140

Tyr Lys Asp Lys Val Tyr Leu Asn Val Thr Thr Asp Asn Thr Ser Leu
145                 150                 155                 160

Asp Asp Phe His Val Asn Gly Gly Glu Leu Ile Leu Ile His Gln Asn
                165                 170                 175

Pro Gly Glu Phe Cys Val Leu
            180


<210>    6
<211>    552
<212>    DNA
<213>    Artificial Sequence

<220>
<223>    nucleotide sequence of OX40L
```

<400> 6
atggaacggg tgcaacctct ggaggagaat gtgggcaatg cagcccggcc aagatttgag 60

aggaacaagc tactgctggt ggcctctgtg atacagggac tggggctcct cctgtgcttc 120

acctacatct gcctgcactt cagcgctctt caggtgtcac accggtatcc ccgcatacaa 180

agtatcaagg tccagtttac cgagtataag aaagagaagg gtttcatcct cactagtcag 240

aaggaggatg aaatcatgaa ggtgcagaac aactcagtta ttattaactg tgacggcttt 300

tatctgatca gcctgaaggg ctacttctcc caggaagtaa acatcagcct gcattaccag 360

aaggatgagg agcccctgtt tcagctgaag aaggtccggt ccgtgaactc ccttatggtc 420

gcctctctga cgtacaagga caaggtgtac ctcaatgtga ccacagacaa tacatccctg 480

gatgacttcc atgtgaatgg cggagaactg attctgattc accagaaccc tggggagttc 540

tgtgtcttgt ga 552

<210> 7
<211> 705
<212> DNA
<213> Homo sapiens

<400> 7
atgagcactg aaagcatgat ccgggacgtg gagctggccg aggaggcgct ccccaagaag 60

acagggggc cccagggctc caggcggtgc ttgttcctca gcctcttctc cttcctgatc 120

gtggcaggcg ccaccacgct cttctgcctg ctgcactttg gagtgatcgg cccccagagg 180

gaagagttcc ccagggacct ctctctaatc agccctctgg cccaggcagt cagatcatct 240

tctcgaaccc cgagtgacaa gcctgtagcc catgttgtag caaaccctca agctgagggg 300

cagctccagt ggctgaaccg ccgggccaat gccctcctgg ccaatggcgt ggagctgaga 360

gataaccagc tggtggtgcc atcagagggc ctgtacctca tctactccca ggtcctcttc 420

aagggccaag gctgcccctc cacccatgtg ctcctcaccc acaccatcag ccgcatcgcc 480

gtctcctacc agaccaaggt caacctcctc tctgccatca agagcccctg ccagagggag 540

accccagagg gggctgaggc caagccctgg tatgagccca tctatctggg aggggtcttc 600

cagctggaga agggtgaccg actcagcgct gagatcaatc ggcccgacta tctcgacttt 660

gccgagtctg ggcaggtcta ctttgggatc attgccctgt gagga 705

<210> 8
<211> 233
<212> PRT
<213> Artificial Sequence

<220>
<223> amino acid seqeunce for mutated TNF alpha (mTNF-a)

<400> 8

```
Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Pro Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230
```

<210> 9
<211> 702
<212> DNA
<213> Artificial Sequence

<220>
<223> nucleotide sequence of mutated TNF alpha (mTNF-a)

<400> 9

```
atgagcactg aaagcatgat ccgggacgtg gagctggccg aggaggcgct ccccaagaag     60

acagggggc cccagggctc caggcggtgc ttgttcctca gcctcttctc cttcctgatc     120

gtggcaggcg ccaccacgct cttctgcctg ctgcactttg gagtgatcgg cccccagagg    180
```

gaagagttcc ccagggacct ctctctaatc agccctctgg cccagccggt cagatcatct 240

tctcgaaccc cgagtgacaa gcctgtagcc catgttgtag caaaccctca agctgagggg 300

cagctccagt ggctgaaccg ccgggccaat gccctcctgg ccaatggcgt ggagctgaga 360

gataaccagc tggtggtgcc atcagagggc ctgtacctca tctactccca ggtcctcttc 420

aagggccaag gctgcccctc cacccatgtg ctcctcaccc acaccatcag ccgcatcgcc 480

gtctcctacc agaccaaggt caacctcctc tctgccatca agagcccctg ccagagggag 540

accccagagg gggctgaggc caagccctgg tatgagccca tctatctggg aggggtcttc 600

cagctggaga agggtgaccg actcagcgct gagatcaatc ggcccgacta tctcgacttt 660

gccgagtctg ggcaggtcta ctttgggatc attgccctgt ga 702

<210> 10
<211> 46
<212> DNA
<213> Artificial Sequence

<220>
<223> Forward primer for 4-1BBL (PCR)

<400> 10
tctagagcta gcgaattcgc caccatggaa tacgcctctg acgctt 46

<210> 11
<211> 38
<212> DNA
<213> Artificial Sequence

<220>
<223> Reverse primer for 4-1BBL (PCR)

<400> 11
ttcgcggccg cggatcctta ttccgacctc ggtgaagg 38

<210> 12
<211> 40
<212> DNA
<213> Artificial Sequence

<220>
<223> Forward primer for mbIL-21 (PCR)

<400> 12
tagagctagc gaattcgcca ccgccaccat ggctctgccc 40

<210> 13
<211> 35
<212> DNA
<213> Artificial Sequence

<220>
<223> Reverse primer for mbIL-21 (PCR)

<400> 13
tcgcggccgc ggatcctcaa tacagggtga tgacc 35

<210> 14
<211> 38
<212> DNA
<213> Artificial Sequence

<220>
<223> Forward primer for OX40L (PCR)

<400> 14
tagagctagc gaattcgcca ccatggaacg ggtgcaac 38

<210> 15
<211> 37
<212> DNA
<213> Artificial Sequence

<220>
<223> Reverse primer for OX40L (PCR)

<400> 15
tcgcggccgc ggatcctcac aagacacaga actcccc 37

<210> 16
<211> 40
<212> DNA
<213> Artificial Sequence

<220>
<223> Forward primer for mTNF-a (PCR)

<400> 16
tagagctagc gaattcgcca ccgccaccat ggctctgccc 40

<210> 17
<211> 34
<212> DNA
<213> Artificial Sequence

<220>
<223> Reverse primer for mTNF-a (PCR)

<400> 17
tcgcggccgc ggatcctcac agggcaatga tccc 34

<210> 18
<211> 20
<212> DNA

<213> Artificial Sequence

<220>
<223> Forward primer for 4-1BBL (RT-qPCR)

<400> 18
tctgagacag ggcatgtttg 20

<210> 19
<211> 20
<212> DNA
<213> Artificial Sequence

<220>
<223> Reverse primer for 4-1BBL (RT-qPCR)

<400> 19
ccaccagttc tttggtgtcc 20

<210> 20
<211> 20
<212> DNA
<213> Artificial Sequence

<220>
<223> Forward primer for mTNF-a (RT-qPCR)

<400> 20
aacctcctct ctgccatcaa 20

<210> 21
<211> 20
<212> DNA
<213> Artificial Sequence

<220>
<223> Reverse primer for mTNF-a (RT-qPCR)

<400> 21
atagtcgggc cgattgatct 20

<210> 22
<211> 20
<212> DNA
<213> Artificial Sequence

<220>
<223> Forward primer for mbIL-21 (RT-qPCR)

<400> 22
tggaaacaat gagcgaatca 20

<210> 23

<211> 20
<212> DNA
<213> Artificial Sequence

<220>
<223> Reverse primer for mbIL-21 (RT-qPCR)

<400> 23
aaccgctcca ggaactcttt 20

<210> 24
<211> 20
<212> DNA
<213> Artificial Sequence

<220>
<223> Forward primer for hTOP1 (RT-qPCR)

<400> 24
ccagacggaa gctcggaaac 20

<210> 25
<211> 22
<212> DNA
<213> Artificial Sequence

<220>
<223> Reverse primer for hTOP1 (RT-qPCR)

<400> 25
gtccaggagg ctctatcttg aa 22